# EUROPEAN PATENT SPECIFICATION

(11) **EP 4 065 100 B1**
(45) Date of publication and mention of the grant of the patent: **08.05.2024**
(21) Application number: 19958146.3
(22) Date of filing: 30.12.2019
(51) Int. Cl.: A61K 31/404, A61P 35/00, A61P 25/20, A61K 31/33, A61K 45/06, A61P 43/00, G01N 33/00

(54) **DESTABILIZER OF CRY1 FOR THE TREATMENT OF CIRCADIAN RHYTHM ASSOCIATED DISEASES AND DISORDERS**
DESTABILISATOR VON CRY1 ZUR BEHANDLUNG VON BIORHYTHMUSBEDINGTEN ERKRANKUNGEN UND STÖRUNGEN
DÉSTABILISANT DU CRY1 POUR LE TRAITEMENT DE MALADIES ET DE TROUBLES ASSOCIÉS AU RYTHME CIRCADIEN

(43) Date of publication of application: 05.10.2022
(73) Proprietor: KOC Universitesi, 34450 Istanbul (TR)
(72) Inventor: KAVAKLI, Ibrahim Halil, 34450 Sariyer/Istanbul (TR); TURKAY, Metin, 34450 Sariyer/Istanbul (TR); GUL, Seref, 34450 Sariyer/Istanbul (TR)
(74) Representative: Dericioglu, E. Korhan
(86) International application number: PCT/TR2019/051248
(87) International publication number: WO 2021/137771

(56) References cited:
- EP-A1- 2 408 906
- WO-A1-2010/108195
- WO-A1-2018/132383
- Saran Saran Anand Anand R R ET AL: "JMJD5 links CRY1 function and proteasomal degradation", PLoS Biology, 30 November 2018 (2018-11-30), pages 1-23, XP093046109, United States DOI: 10.1371/journal.pbio.2006145 Retrieved from the Internet: URL:https://journals.plos.org/plosbiology/ article/file?id=10.1371/journal.pbio.20061 45&type=printable [retrieved on 2023-05-11]
- CHA Hyo Kyeong, Chung Sooyoung, Lim Hye Young, Jung Jong-Wha, Son Gi Hoon: "Small Molecule Modulators of the Circadian Molecular Clock With Implications for Neuropsychiatric Diseases", Frontiers in Molecular Neuroscience, vol. 11, no. 11, 21 January 2019 (2019-01-21), XP055839069, DOI: 10.3389/fnmol.2018.00496

## Description

### FIELD OF THE INVENTION

The present invention discloses and claims 11-(4-chlorophenyl)-4-(2,3-dihydro-1H-indole-1-carbonyl)-3,11-dimethyl-5,10,dioxatricyclo[7.4.0.0,2,6,]trideca-1,3,6,8-tetraen-13-one (Formula I ) as CRY1-binding small molecule and destabilizer of CRY1, and method of using said compound Formula I for treating circadian rhythm associated diseases, including cancer. Pharmaceutical compositions comprising Formula I and methods for the preparation of formula (I) are also disclosed and claimed.

### BACKGROUND

The circadian clock generates a 24-hour rhythm through which physiology and behavior are adapted to daily changes in the environment. Many biological processes like hormone secretion, and sleep-wake cycles are controlled by the circadian clock. Therefore, an innate malfunctioning of the circadian clock and the related pathways can cause various pathologies. Sleep disorders, altered metabolism, obesity, diabetes, mood disorders, cancer, and cardiovascular diseases are all linked with abnormal circadian rhythm.

At the molecular level, the clockwork of the cell involves several proteins that participate in positive and negative transcriptional/translational feedback loops (TTFL). BMAL1 and CLOCK are transcription factors that bind E-box elements (CACGTG) in clock-controlled genes including Period and Cryptochrome and thereby exert a positive effect on circadian transcription. The mammalian PERIOD (PER) and CRYPTOCHROME (CRY) proteins form heterodimers that interact with casein kinase Iε (CKIε) and then translocate into nucleus where CRY acts as a negative regulator of BMAL1/CLOCK-driven transcription. Upon phosphorylation CRYs are ubiquitinated by E3 ubiquitin ligases e.g. FBXL3 and FBXL21 and directed to proteasome for the degradation. FBXL21 and FBXL3 act antagonistically on CRY to regulate its stability differentially in the cytosol and nucleus, respectively. The FBXL3 protein participates in the negative feedback loop by binding to the CRY1 and CRY2 proteins to facilitate their polyubiquitination and their subsequent degradation by the proteasome (Hirano et al., 2013; Yoo et al., 2013).

The crystal structure of the CRY-FBXL3 revealed the critical region (FAD binding pocket) on CRY for the FBXL3 interaction (Xing et al., 2013).

Since the circadian system regulates several aspects of our physiology, it is not surprising that disturbed circadian rhythm can lead into diseases in human. It is, therefore, essential to find small molecules to correct disturbed circadian rhythm. There are several studies have been carried out to find small molecules affect circadian rhythm based on phenotypic changes in the circadian rhythm of reporter cells using high-throughput screening assay. These studies result in identification several molecules affect different features of circadian rhythm (Chen et al., 2012; Hirota et al., 2012; Hirota et al., 2008; Lee and Sancar, 2011). For example a molecule (named as GSK4112) was shown to enhance REV-ERB's repressor function toward Bmal1 transcription and greatly altered circadian clock and metabolism (Solt et al., 2012). Another example, identification of KL001 molecule, increases stability of the CRYs and suppresses the gluconeogenesis (Hirota et al., 2012). Alternatively, structure-based drug design approach can be utilized to design small molecules by using the available crystal structure core clock proteins (Czama et al., 2013; Schmalen et al., 2014; Xing et al., 2013).

Given the increasing prevalence of circadian disruptions and its deleterious health consequences, it is important to prevent and minimize circadian disruptions in our daily life and their detrimental effects. The study of fast, dynamic biological processes demands the development of approaches that conditionally and selectively modulate a specific aspect of a gene product in a rapid and tunable manner. In addition, all information regarding how clock-work is regulated at the molecular level comes from genetics studies. In spite of the specificity and robustness of genetic manipulation, the lack of temporal control makes genetic approaches problematic for the study of biological processes occurring on the millisecond to minute time scale (e.g., posttranslational modification, signal transduction) (Lampson and Kapoor, 2006). Treatment strategies involving genetic or alterations in a patient's daily behavior are clearly not ideal for many reasons. A more promising therapeutic approach would be to use a pharmaceutical agent that selectively targets the molecular clock. These challenges will be overcome by discovering the small molecules that specifically bind and regulate the activity of each of these core clock proteins. Currently, a growing number of groups are looking for interventions ranging from modulating environmental cues to manipulation of molecular machinery. Therefore, molecules that either bind to modifiers of core clock proteins, such as casein kinase Iε (CK1ε), glycogen synthase kinase 3 β (GSK3β), AMP-activated protein kinase (AMPK), and SIRT1 with varying effect on the speed of the clock and amplitude; or that target CRY (Chun et al., 2014; Oshima et al., 2015), REV-ERBs, and RORs, three of core clock proteins, were identified from high-throughput screening.

An effective method to identify small molecules that perturb a biological system is structure-based design. Since the crystal structures of clock proteins are now available, this approach can lead to identification of clock modulating compounds targeting clock proteins.

Patent document WO2010108195 concerns the use of agonists and antagonists of AMP-activated protein kinase (AMPK) for modulating circadian rhythms. More particularly, the disclosure provides compositions and methods for screening and modulating sleep behavior.

Anand R. Saran et al. reported that genetic deletion of JMJD5 results in greater CRY1 stability, reduced CRY1 association with the proteasome, and disruption of circadian gene expression. Further, they reported that in the absence of JMJD5, AMP-regulated protein kinase (AMPK)-induced CRY1 degradation is impaired, establishing JMJD5 as a key player in this mechanism. JMJD5 cooperates with CRY1 to repress circadian locomotor output cycles protein kaput (CLOCK)-brain and muscle ARNT-like protein 1 (BMAL1), thus linking CRY1 destabilization to repressive function. In addition, they found that ablation of JMJD5 impacts FBXL3- and CRY1-related functions beyond the oscillator (Saran, Anand R., et al. "JMJDS links CRY1 function and proteasomal degradation." PLoS Biology 16.11 (2018): e2006145).

In the European patent document EP2408906A1, the role of AMPK in circadian rhythms and methods of screening for small molecules that modulate such rhythms are disclosed. The disclosure demonstrates that AMPK phosphorylates the transcription repressor CRY1 and CRY2 and stimulates their proteasomal degradation. According to this invention, the use of an AMP kinase agonist or antagonist are disclosed for the manufacture of a medicament to modulate circadian rhythms in a subject.

Many physiological variables require a robust circadian clock for their proper function. There is a need for circadian rhythm controlling molecules and compositions as potential treatments for clock-related diseases, including cancer. It has therefore become increasingly interesting to identify small molecules that can specifically modulate regulatory core clock proteins since they have the potential to manage these diseases.

Cryptochrome 1 (Cry1), one of the key circadian clock genes, plays an important role in circadian clock and clock-related diseases. Previous researches have confirmed that the dysregulation of Cry 1 correlates with the occurrence and progression of many types of cancer. The abnormal expression of the core circadian clock gene Cryptochrome 1 (Cry1) was found in many types of cancers. (Gauger et al., 2005; Kelleher et al., 2014; Hongyan et al., 2013). It was found that Cry1 regulates clock gene network and promotes proliferation and migration in a Akt dependent manner in human osteosarcoma cells (Lei Zhou et al., 2018). Cry1 or CRY1 may be a prognostic biomarker and a promising therapeutic target for treatment of cancer.

Despite advances in drug discovery directed to identifying therapeutic small molecules for CRY1 binding, there is still a scarcity of compounds that are both potent, efficacious, and selective destabilizer of CRY1 protein. Furthermore, there is a scarcity of compounds effective in the treatment and/or prevention of disorders associated with the circadian rhythm. These needs and other needs are satisfied by the present invention.

### SUMMARY OF THE INVENTION

According to a first aspect of the invention there is provided a CRY1-binding compound of Formula I or pharmaceutically acceptable salts thereof.

Accordingly, a broad embodiment of the invention is directed to a CRY1-binding compound of Formula I :

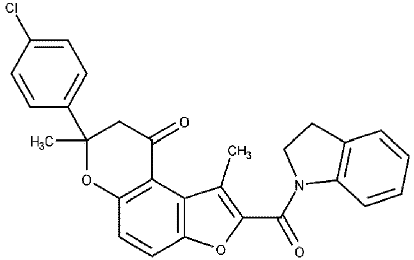

The other aspect of the present invention is to provide a CRY1-binding compound for treating and/or preventing a circadian rhythm associated diseases or disorders, including cancer, wherein the compound is characterized by destabilizing of CRY1.

In a further aspect, the present invention relates to a CRY1-binding compound of the invention for use in enhancing the degradation of CRY1 in a mammal.

Another aspect of the present invention is to provide a CRY1-binding compound capable of controlling circadian rhythm via increasing period length and dampening circadian rhythm amplitude.

Another embodiment of the present invention relates to a method for identifying a compound for destabilizing the CRY1 protein.

The invention can be used for the preparation of a medicament useful in the treatment and/or prevention of disorders due to CRY1. Yet another objective of the present invention is to provide a pharmaceutical composition comprising a pharmaceutical carrier and a therapeutically effective amount of a compound of Formula I, or a pharmaceutically acceptable salt thereof.

This object and other objects of this invention become apparent from the detailed discussion of the invention that follows.

### Brief Description of Figures

The present invention is illustrated in the accompanying figures wherein;

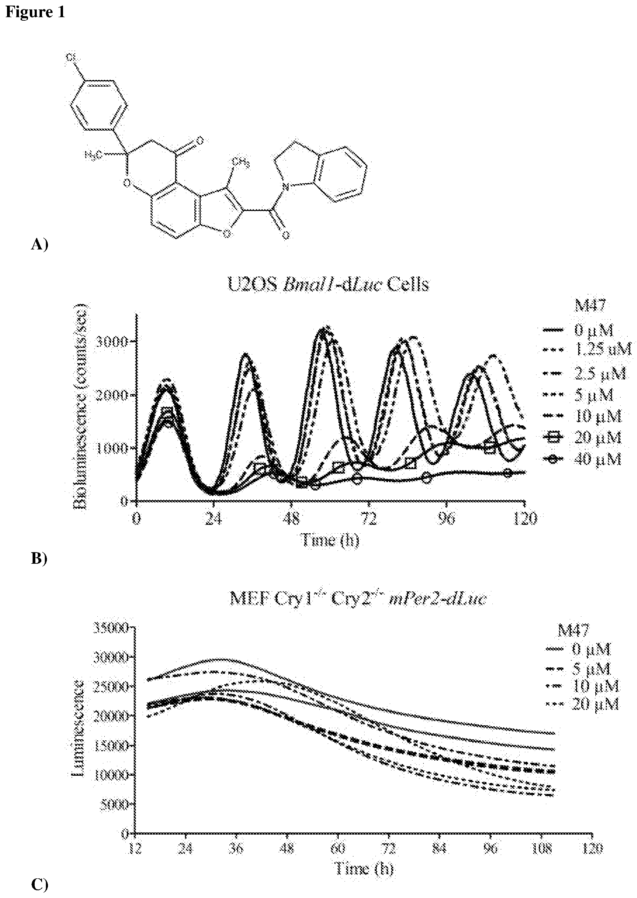

Figure 1 is an illustration of FORMULA I changes period of U2-OS cells dose dependently. (A) Structure of FORMULA I (B) Representative figure for the effect of FORMULA I on the bioluminescence rhythm of U2-OS cells stably expressing Bmal1-dLuc. FORMULA I lengthened the circadian rhythm dose dependently (Data represent the mean ± SEM, n=4 with duplicates ***: p < 0.001 **: p < 0.005, *: p < 0.05 versus DMSO control by student t-test). (C) Effect of FORMULA I on the Cry1^{-/-}/Cry2^{-/-} mouse embryonic fibroblasts transiently transfected with mPer2-dLuc reporter.

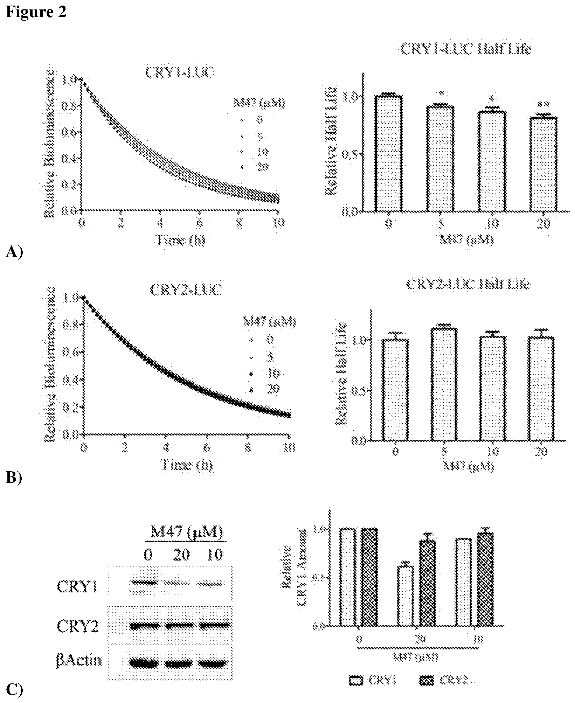

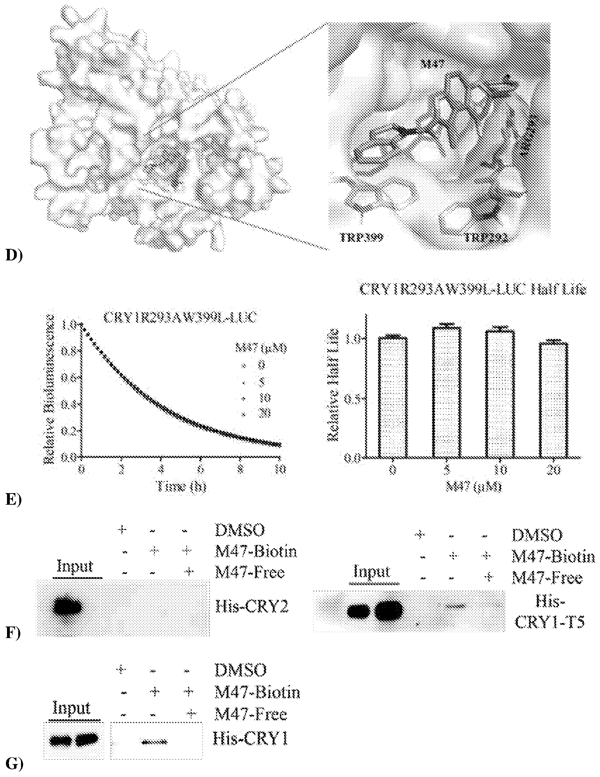

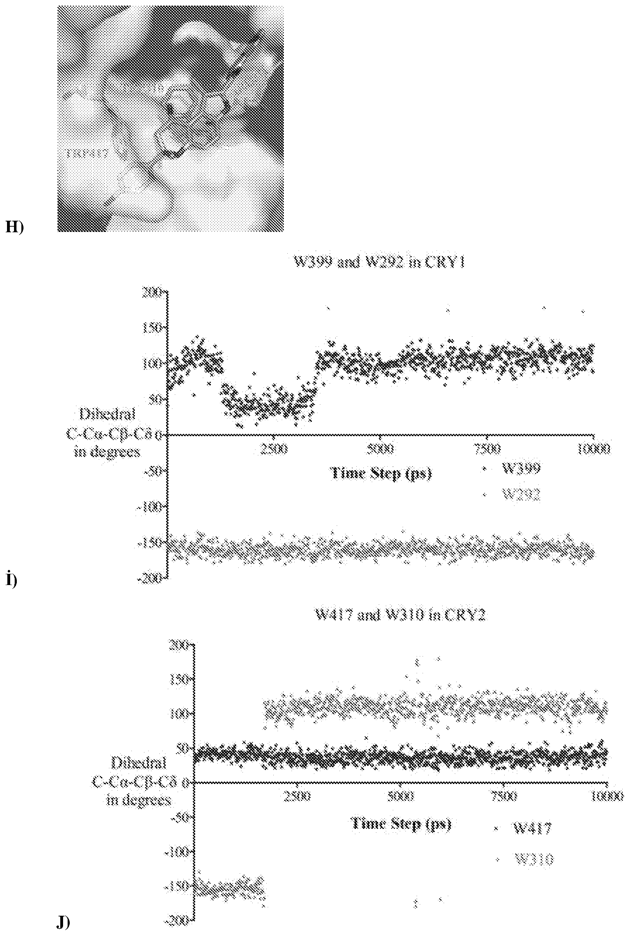

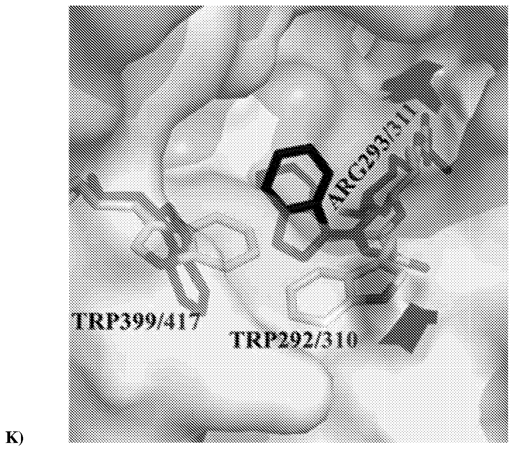

Figure 2 is an illustration of FORMULA I decreases the half-life of CRY1. (A) FORMULA I increased the degradation rate of CRY1-LUC dose dependently. HEK 293T cells were transfected with Cry1-Luc plasmid. 24h after transfection cells were treated with different doses of FORMULA I or solvent (DMSO final 0.5%) as control. 24h after molecule treatment cells were treated with cycloheximide (20µg/ml final) and bioluminescence was recorded. Normalized half-life is shown with ± SEM (n=4 with triplicates). *p<0.05 **p<0.01 (B) FORMULA I did not affect the degradation rate of CRY2-LUC (n=4 with triplicates). (C) FORMULA I decreased the steady-state level of CRY1 in U2-OS cells. CRY1 level relative control treated with solvent (DMSO) was shown ± SEM (n=3). (D) Binding pose of FORMULA I on the equilibrated CRY1 (4K0R) structure predicted by AutodockVina with -13.4kcal/mol binding energy. Protein structure is shown in surface. FBXL3 binding residues (4I6J) are colored as red, PER2 binding residues (4CT0) are colored as orange. FORMULA I interacting residues are shown in sticks (carbons are white, nitrogens are blue, oxygens are red, however, carbons in FORMULA I are shown in gray). (E) FORMULA I did not affect the half-life of mutant CRY1R293AW399L-LUC degradation (Data shown with ± SEM n=3 with triplicates). (F,G) HEK293T cells transfected with Cry 1-His-Myc, Cry2-His-Myc, or Cty1-T5-His-Myc plasmids then lysates were subjected to pull-down assay. Lysates treated with solvent (DMSO), 50µM bFORMULA I, and 50µM bFORMULA I with 100µM FORMULA I (competitor). While FORMULA I binds to PHR of CRY1 (F, G) it does not bind to CRY2 (F) (n=3). (H) Binding mode of FORMULA I on equilibrated CRY2 (4I6G) predicted by AutodockVina with binding energy -9.4kcal/mol. W310 occupies the binding region of FORMULA I. Coloring is done as explained in (D) with modifications. Carbon atoms of CRY2 are shown in cyan. (I) Dihedral angle between C-Cα-Cβ- Cδ atoms of W399 and W292 in CRY1; W417 and W310 in CRY2 throughout the simulations. Dihedral angle around -1500 of W292 in CRY1, W310 in CRY2 represents the parallel position; angle > 00 represents the perpendicular position of these residues to R293 and R311 in CRY1 and CRY2, respectively. Similarly, dihedral angle around 900 of W399 in CRY1, W417 in CRY2 represents the parallel position; angle around 400 represents the perpendicular position of these residues. (K) Superimpose image of equilibrated CRY1 and CRY2 structures. FORMULA I binding residues (W399, W292, R293) in CRY1 and corresponding residues in CRY2 (W417, W310, R311) are shown in sticks. Coloring is done as explained in (H).

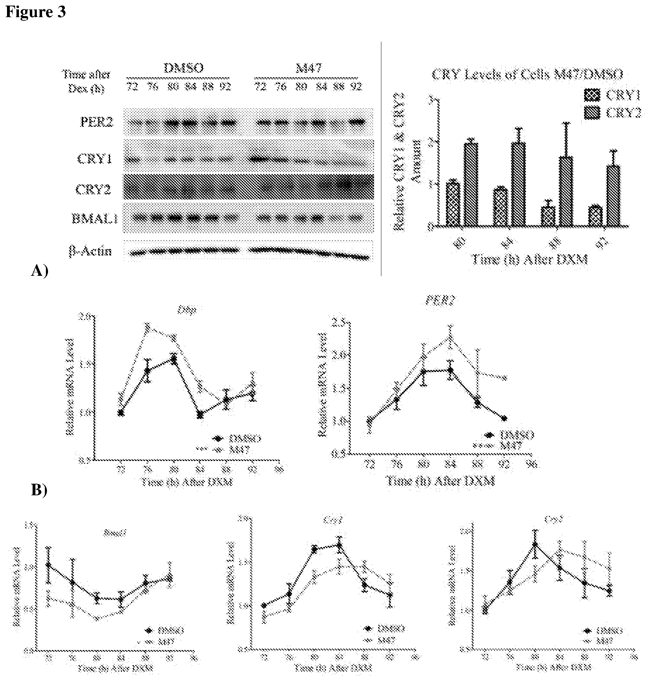

Figure 3 is an illustration of FORMULA I decreased CRY1 level in synchronized U2-OS cells. Confluent U2-OS cells were synchronized by 2h treatment with dexamethasone (0.1µM) and medium replaced with fresh medium containing FORMULA I or solvent (DMSO final 0.5%). Cells were harvested at indicated time points. (A) Lysed cells were analyzed via protein immunoblot technique. FORMULA I decreased the protein level of CRY1 and increased the level of CRY2 between 80th -92nd h. CRY levels in DMSO control divided by that of in FORMULA I treated samples were reported (mean ± SEM, n=4). (B) Cells were subjected to reverse-transcription-quantitative polymerase chain reaction (RT-qPCR). FORMULA I treatment changed the peak time of Dbp, Cry1 and Cry2, in addition, increased the level of Dbp and Per2. Expression level of genes was normalized according to the expression level of that gene at 72ndh. (Data represent the mean ± SEM, n=3 with duplicates * * *: p < 0.001 **: p < 0.005 , * : p < 0.05 versus DMSO control by two-way ANOVA).

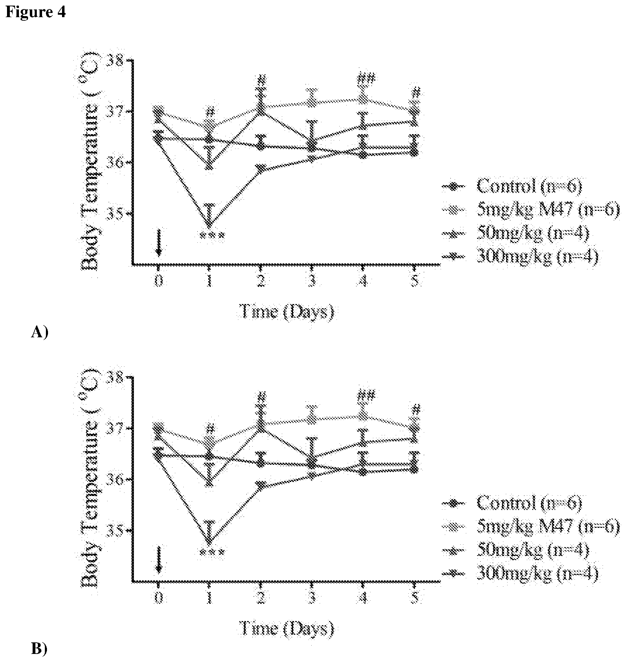

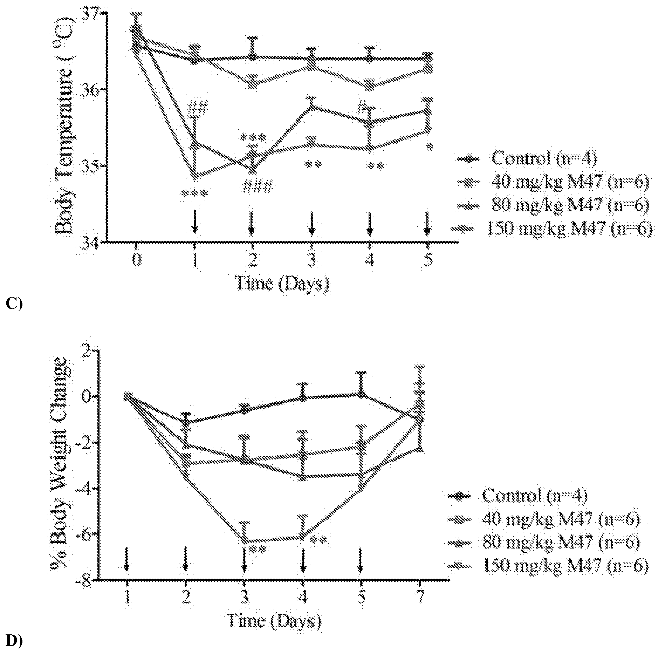

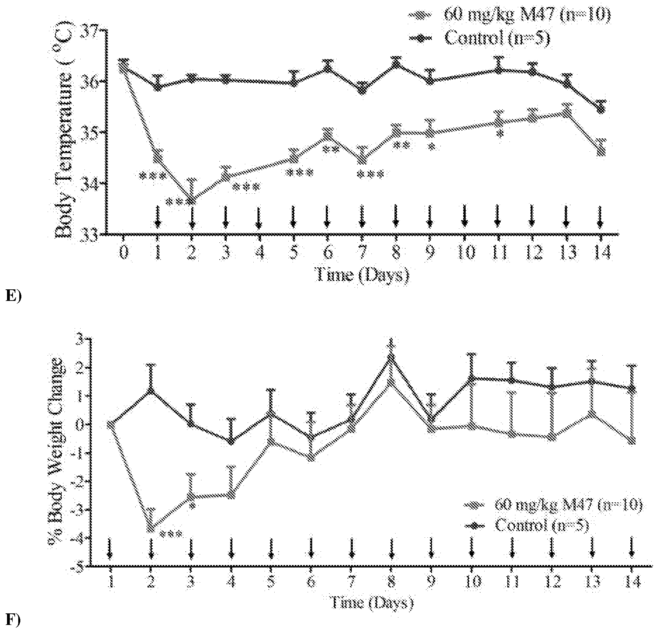

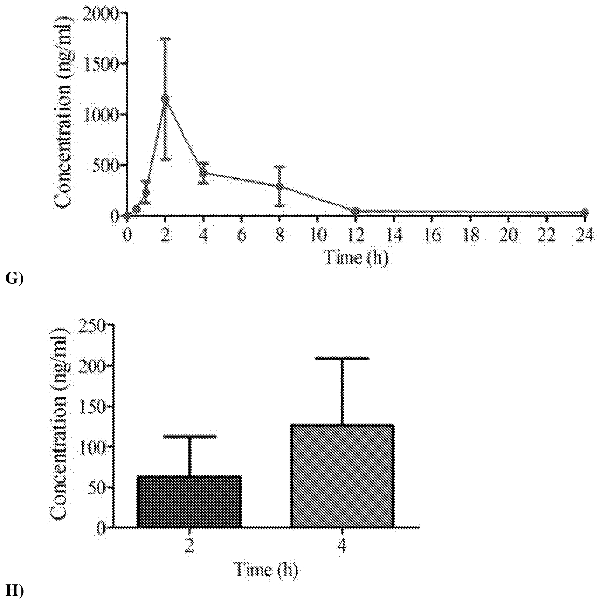

Figure 4 is an illustration of toxicity and pharmacokinetics of FORMULA I in mice. (A) Body temperatures in C57BL/6J mice treated with single intraperitoneal doses of 5 mg/kg, 50 mg/kg, 300 mg/kg FORMULA I or vehicle during 15-day observation period. Body temperatures (°C) were expressed as mean ± SEM. "↓" indicates the treatment days of FORMULA I. ***p<0.001, control vs 300 mg/kg FORMULA I ; #p<0.05, ##p<0.01 control vs 5 mg/kg FORMULA I (Two-way ANOVA with Bonferroni post hoc test). (B) Body weight changes (%) in C57BL/6J mice treated with single intraperitoneal doses of 5 mg/kg, 50 mg/kg, 300 mg/kg FORMULA I or vehicle during 15-day observation period. Body weight changes (%) were expressed as mean ± SEM. "↓" indicates the treatment days of FORMULA I . *p<0.05,**p<0.01, ***p<0.001, control vs 300 mg/kg FORMULA I ; #p<0.05, ##p<0.01, ###p<0.001 5 mg/kg vs 300 mg/kg FORMULA I ; jp<0.05, jjp<0.01 control vs 50 mg/kg; kp<0.05, 5 mg/kg vs 50 mg/kg FORMULA I (Two-way ANOVA with Bonferroni post hoc test). (C) Body temperatures in C57BL/6J mice treated with intraperitoneal doses of 40 mg/kg, 80 mg/kg, 150 mg/kg FORMULA I or vehicle for 5 days. Body temperatures (°C) were expressed as mean ± SEM. "↓" indicates the treatment days of FORMULA I . *p<0.05,**p<0.01, ***p<0.001, control vs 150 mg/kg FORMULA I ; #p<0.05, ##p<0.01, ###p<0.001 control vs 80 mg/kg FORMULA I (Two-way ANOVA with Bonferroni post hoc test). (D) Body weight changes (%) in C57BL/6J mice treated with intraperitoneal doses of 40 mg/kg, 80 mg/kg, 150 mg/kg FORMULA I or vehicle for 5 days. Body weight changes (%) were expressed as mean ± SEM. "↓" indicates the treatment days of FORMULA I . **p<0.01 control vs 150 mg/kg FORMULA I (Two-way ANOVA with Bonferroni post hoc test). (E) Body temperatures in C57BL/6J mice treated with intraperitoneal dose 60 mg/kg FORMULA I or vehicle for 14 days. Body temperatures (°C) were expressed as mean ± SEM. "↓" indicates the treatment days of FORMULA I. *p<0.05, **p<0.01, ***p<0.001, control vs 60 mg/kg FORMULA I (Two-way ANOVA with Bonferroni post hoc test). (F) Body weight changes (%) in C57BL/6J mice treated with intraperitoneal dose of 60 mg/kg FORMULA I or vehicle for 14 days. Body weight changes (%) were expressed as mean ± SEM. "↓" indicates the treatment days of FORMULA I. *p<0.05, ***p<0.001, control vs 60 mg/kg FORMULA I (Two-way ANOVA with Bonferroni post hoc test). (G) Mean plasma concentration-time curve of FORMULA I administered at 100 mg/kg intraperitoneally to C57BL/6J mice. Data were expressed as mean ± SEM (n=4 per time point). (H) FORMULA I levels in brain tissue at 2nd (n=2) and 4th (n=2) hours following FORMULA I administration at a dose of 100 mg/kg intraperitoneally to C57BL/6J mice. Data were expressed as mean ± SEM.

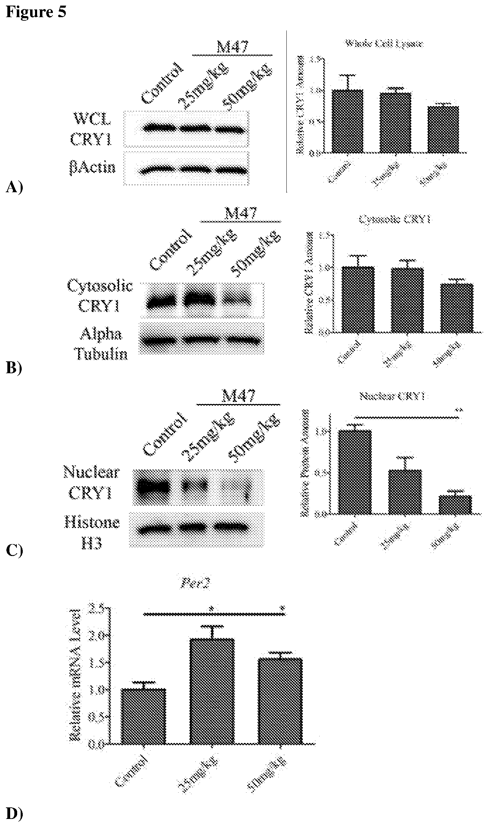

Figure 5 is an illustration of FORMULA I decreased the nuclear CRY1 in mice liver. FORMULA I (25 mg/kg or 50mg/kg) or vehicle intraperitoneally administered to C57BL/6J mice. 6h after the treated mice were sacrificed (n=3). While FORMULA I slightly decreased the CRY1 levels in whole cell lysate (WCL) and cytosolic fraction, decreased the nuclear CRY1 level significantly. Effect of FORMULA I in (A) WCL (B) cytosolic fraction, and (C) nuclear fraction of mice liver. (D) Liver samples subjected to RT-PCR. FORMULA I treatment increased the mRNA level of Per2. (Data represent the mean ± SEM, n=3 (with duplicates in RT-PCR) *: p < 0.05 versus control by student t-test).

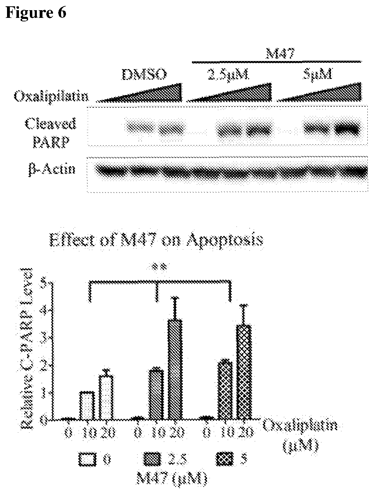

Figure 6 is an illustration of FORMULA I enhanced the effect of oxalipilatin in p53 null MSF cells. Ras pT24 transformed p53 null MSF fibroblast cells were treated with 0, 10 or 20µM oxaliplatin and incubated for 24h. Then either DMSO or FORMULA I was added and incubated for 16h. Cells were lysed and analyzed via protein immunoblot technique. At each cases FORMULA I increases the cleaved PARP protein level. Bar graph was drawn normalizing to 10µM dosage of oxaliplatin (Data represent the mean ± SEM, n=4 **: p < 0.005, * : p < 0.05 versus DMSO control by two-way ANOVA).

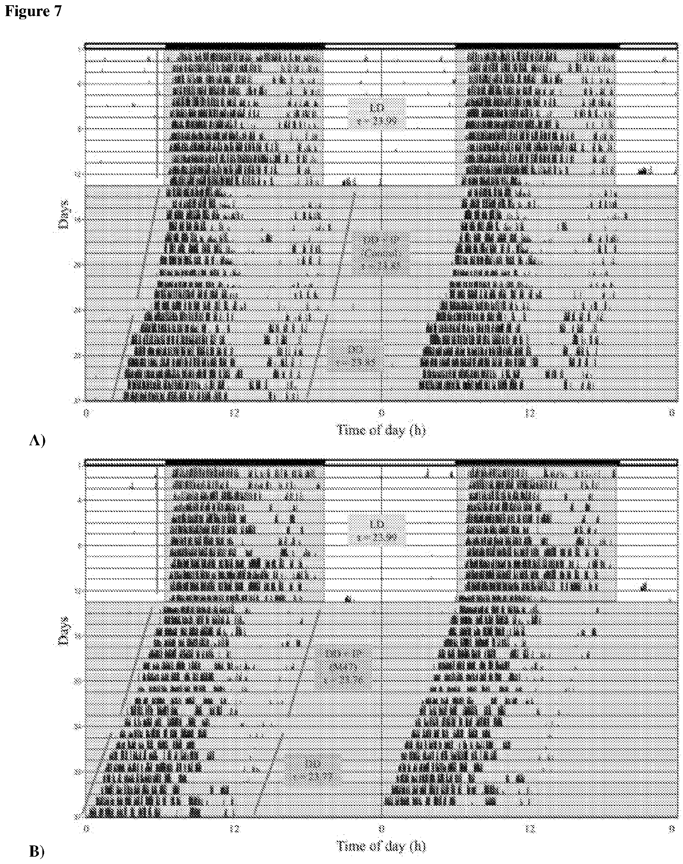

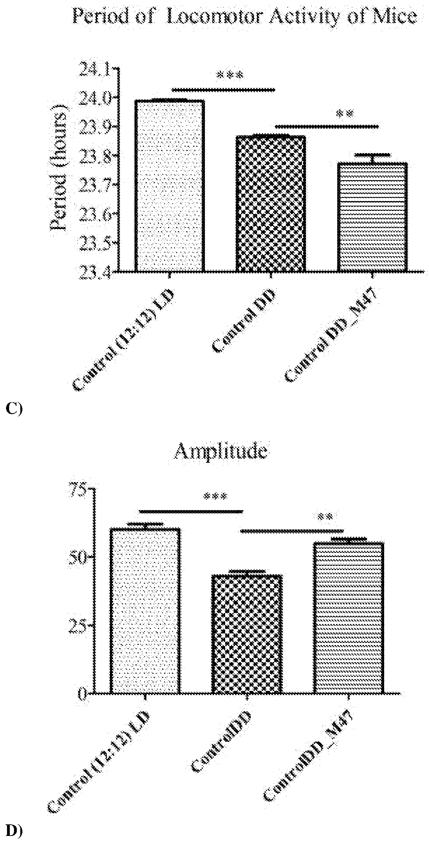

Figure 7 Figure 7 is an illustration of FORMULA I shortened the period of locomotor activity in mice. C57BL/6J mice were synchronized 10 days under 12h: 12h light-dark cycles. After 10 days mice were treated with intraperitoneal dose of 60 mg/kg FORMULA I or vehicle for 14 days and kept under constant darkness (n=12). Locomotor activity of mice were recorded for additional 10-days without any injection. During these days locomotor activity of mice were recorded by ClockLab (Actimetrics) software program. A) Representative locomotor activity of mice injected with vehicle (n=5) or B) FORMULA I (n=7). C) Period and D) amplitude of mice under different conditions and injected with vehicle or FORMULA I. Period and amplitude values were calculated by using ClockLab Analysis (Actimetrics) program. Statistical significance was evaluated using oneway analysis of variance (ANOVA), followed by a Tukey's multiple comparisons test using Prism software (GraphPad Software)

### DETAILED DESCRIPTION OF THE INVENTION

In the present invention, it is aimed to discover a novel CRY1-binding small compound using a structure-based approach. As a result, the interaction of a CRY1-binding compound with CRY1 has been predicted in *silico* and then demonstrated experimentally.

The CRY1-binding compound was identified that destabilizes CRY1 protein and enhances the degradation of CRY1 both in *vivo* and in *vitro.*

Furthermore, it was discovered that, as a result of this interaction, the subsequent degradation of CRY1 by proteasome is regulated by the CRY1-binding compound resulting in suppression of the CRY1 function thereof. A decrease in nuclear CRY1 level leads to the participation of the negative arm of the TTFL and, in turn, and dampen the amplitude of the circadian rhythm with increase in period length.

The present invention relates to a CRY1-binding compound (Formula I).

Unless specified otherwise, the term "Formula I" or "compound" or "FORMULA I" refers to compounds of Formula I, prodrugs thereof, salts of the compound and/or prodrug, hydrates or solvates of the compound, stereoisomers, tautomers, isotopically labeled compounds, and polymorphs.

It is an object of this invention to provide a CRY1-binding compound having the chemical name 11-(4-chloroplienyl)-4-(2,3-dihydro-1H-indole-1-carbonyl)-3,11-dimethyl-5,10,dioxatricyclo[7.4.0.0,2,6,]trideca-1,3,6,8-tetraen-13-one (IUPAC) as destabilizer of CRY1 and so mediator of CRY1 degradation by proteasome.

In accordance with the purpose(s) of the invention, as embodied and broadly described herein, the present invention relates to a CRY1-binding compound, capable of destabilizing CRY1 by binding to FAD-binding pocket of CRY1. In this regard, the invention relates to a compound having the following Formula I: 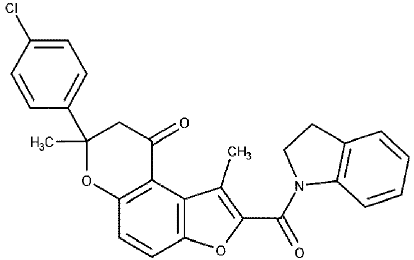 or a pharmaceutically acceptable salt thereof.

In a further aspect, the present invention relates to a compound (Formula I) that binds to a CRY1 protein and negatively modulate CRY1 activity.

The present invention relates to a novel CRY1-binding small molecule that affects the degradation of CRY1 protein in the nucleus through the destabilization of the CRY1 and thus dampen the circadian rhythm. FAD plays a time and locationdependent role to regulate CRY stability by competing with FBXL3, which is supported by the fact that FBXL3 is predominantly localized in the nucleus (Godinho et al., 2007; Hirano et al., 2013; Yoo et al., 2013). When Formula I is present, it binds to CRY1 via FAD binding packet which is critical for the FBXL3 interaction and enhance the degradation of CRY1 by proteasome. Upon binding of Formula I to CRY1, the amount of the CRY1 in the nucleus abolished. Thus, Formula I dampen the amplitude of the circadian rhythm. Formula I exhibits period-lengthening activities by binding to the FAD-biding pocket of CRY1 in competition with FBXL3.

In one embodiment of the invention, the disclosed compound exhibits selectivity and high affinity for the CRY1 protein. Thus, the interaction is potent.

Cryptochromes (CRY1, CRY2) proteins have the characteristics of an N-terminal photolyase homology (PHR) domain. The PHR domain can bind to the flavin adenine dinucleotide (FAD) cofactor and a light-harvesting chromophore. The structure of CRY1 is characterized by an α/β domain and a helical domain (Lin and Todo, 2005).

In certain aspects, the compound binds the PHR region of CRY1 which is a critical domain for the rhythmicity of the cells (Khan et al., 2012). Docking pose and mutational analysis show that the compound binds to W399 in FAD binding pocket and R293 which connects FAD binding pocket to the secondary pocket. In another embodiment, binding residues of the compound are R293, W292 and W399 in CRY1. Different conformational preferences allowed the compound to specifically bind to CRY1 but not CRY2 due to the steric hindrance governed by W417 and W310. Yet, results confirm the specific binding of the compound to CRY1.

The compound directly targets the clock protein, CRY1 and lengthens the period of the circadian rhythm in human cell lines. Although period shortening molecules were previously disclosed as CRY destabilizers, GO044, GO200, and GO211 shortened the period while enhancing the CRY stability (Oshima et al., 2015).

Moreover, it is found that the compound affects the amplitude of circadian rhythm with increasing period length by decreasing the amount of CRY1 in the nucleus without affecting the protein levels in negative arm PER2, BMAL1 or CLOCK. According to the invention, the compound dampens circadian rhythm amplitude. Further in vivo studies showed that the compound has half-life of 6h in blood and destabilized CRY1 in a mammal. The compound also reduces half-life of CRY1 and overall CRY1 amount. According to the invention, the compound decreases CRY1 level at certain time points and increases circadian clock output genes, e.g. Per2 and Dbp as expected when repressor capacity is attenuated.

As used herein, the term "destabilize", "destabilization" or "destabilizing" refers to accelerated degradation or reduced half-life of CRY1 in the nucleus. This, in turn, causes the reduction in suppression or repressor activity of CRY1.

The term "a therapeutically effective amount" of a compound of the present invention refers to a non-toxic and sufficient amount of the compound of the present invention that will elicit the biological or medical response of a subject, for example, reduction or inhibition of the protein activity or protein: protein interaction, or ameliorate symptoms, alleviate conditions, slow or delay disease progression, or prevent a disease or disorder, etc.

All of the various embodiments of the present invention as disclosed herein relate to methods of treating and/or preventing various diseases and disorders as described herein. As stated herein the compound used in the method of this invention is capable of destabilizing CRY1 protein. It is known that cryptochromes also affect human health, including cancer, sleep disorders, and many physiological disorders.

The invention further provides methods for the treatment or prevention of circadian rhythm related disorders and diseases. Non-limiting examples of circadian rhythm disorders include aging, sleep disorders, altered metabolism (metabolic syndromes), obesity, diabetes, mood disorders, cancer and cardiovascular diseases. Mood disorders including major depressive disorder, bipolar I disorder; sleep disorders including circadian rhythm sleep disorders such as shift work sleep disorder, jet lag syndrome, advanced sleep phase syndrome, non-24-hour sleep-wake syndrome, irregular sleep-wake rhythm and delayed sleep phase syndrome.

In a preferred embodiment of the invention, the circadian rhythm related disease is cancer.

In another embodiment the compound enhances apoptosis in mammalian cancer cells such as p53^{-/-} MEF cells. The toxicity and pharmacokinetic parameters from mice studies suggested that the compound can be used to improve the effectiveness of cancer treatment related with p53 mutations and other type of diseases related with CRY1.

Importantly, knockdown of the *Crys* in p53^{-/-} mice or cell-lines improved sensitivity to cancer chemotherapy by activating tumor suppressor genes (Lee et al., 2013; Ozturk et al., 2009). In addition, overexpression of Per2 genes in human pancreatic cancer cells prevented cell proliferation, initiated apoptosis and behaved synergistically with cisplatin (Oda et al., 2009) thereby suppressed the tumor growth.

According to the invention, it is found that the compound as CRY1 destabilizer is also Per2 enhancer. In another embodiment of the invention, the compound is used as novel anti-cancer therapeutic agent. Since the compound decreases CRY1 level and enhances the level of Per2 both in cell-line and in vivo, the compound is a useful remedy for cancer by promoting apoptosis.

Moreover, the invention relates to a pharmaceutical composition comprising such compounds, uses and methods of use for such compounds in the treatment and/or prevention of disorders associated with the circadian rhythm. In other embodiment of the present invention, a pharmaceutical composition comprising the compound is useful in the treatment and/or prevention of circadian rhythm related diseases and disorders due the destabilization of CRY1.

The present invention relates to pharmaceutical compositions comprising a pharmaceutical carrier and a therapeutically effective amount of a compound of Formula I, or a pharmaceutically acceptable salt thereof.

The present invention also relates to pharmaceutical compositions to treat and/or prevent a CRY1-mediated disorders and/or diseases, such as cancer related to the circadian rhythm.

In one aspect, the disclosure relates to a method for the manufacture of a medicament for destabilizing CRY1 in a mammal comprising combining a therapeutically effective amount of a disclosed compound with a pharmaceutically acceptable carrier or diluent.

The present invention provides a method for identifying a compound for destabilizing CRY1. The method can be established using systems for pharmaceutical screening that are well known in the art.

In one aspect, the present invention provides a method for identifying a compound that destabilize CRY1, wherein the method comprises contacting a compound with CRY1 protein under conditions allowing for the interaction, and determining whether the compound leads to reduction in CRY1 level by using a system that uses a signal and/or a marker generated by the interaction between the compound and CRY1 to detect presence or absence or change of the signal and/or the marker. The term "signal" as used herein refers to a substance that can be detected directly by itself based on the physical properties or chemical properties thereof. The term "marker" refers to a substance that can be detected indirectly when the physical properties or biological properties thereof are used as an indicator.

These examples are intended to representative of specific embodiments of the invention, and are not intended as limiting the scope of the invention.

### SPECIFIC EMBODIMENTS

In these embodiments, a structure-based design was applied to find small molecules that specifically bind to the CRY1 protein. After identifying candidate molecules by virtual screening, experimental studies lead to discover a compound (Formula I ) that specifically binds to CRY. The compound, named hereafter FORMULA I , enhanced the degradation rate of CRY1 and modulated the period of U2-OS Bmal1-dLuc cells. It destabilizes CRY1 protein by binding to the FAD-binding packet of CRY1 in the nucleus both in *vivo* and in *vitro.* Further studies indicated that Formula I dampens the amplitude of the circadian rhythm at the cellular level by promoting the negative arm of the transcriptional/translational feedback loop with increasing the period length. In addition, pharmacological studies with mice indicated that FORMULA I had no toxic effect with half-life of ~6 h in blood. In addition, subcellular fractionation studies from mice liver indicated that FORMULA I selectively decrease CRY1 level in the nucleus. Furthermore, FORMULA I mediated CRY1 reduction enhanced cisplatin-induced apoptosis in Ras transformed *p53*-null fibroblast cells. Effect of FORMULA I on the oxalipilation-induced apoptosis makes it a very promising agent to treat *p53* mutant dependent forms of cancer and other CCRY1 related dieseases.

### Examples

### Example 1 In silico search for compounds that interact with CRY1

### Molecular Dynamics

The mouse CRY1 (mCRY1) structure (PDB ID: 4K0R) was used to identify small molecules targeting its photolyase (PHR) domain. Before docking studies, molecular simulation was performed on CRY1 to obtain a structure similar to that found under physiological conditions.

Firstly, the structure of CRY1 was solvated in a rectangular box and neutralized with counter ions. Subsequently, the system was minimized, heated up to physiological temperature (310⁰K) and simulated for 10ns. CHARMM-PARAM22 force field was used for the molecular dynamics (MD) simulations. RMSD of backbone atoms showed convergence after initial increase upon minimization. The last frame of the simulation was utilized as the receptor for the docking analysis.

### Docking Setup

More than 8 million small molecules with non-identified functions were used as ligands for the docking. Molecules were filtered according to the following criteria to eliminate non-relevant molecules: molecules should have less than 7 H-bond donor, less than 12 H-bond acceptor, less than 600 Da molecular weight, logP < 7, less than 8 rotatable bonds, at least 3 aromatic rings, and at least 4 rings. Openbabel, Autodock4.2, Autodock Tools4 (Morris et al., 2009)and Autodock (Trott and Olson, 2010) program packages which are free for academic purposes, were utilized to prepare ligands (small molecules) for the docking. Commercially available small molecule libraries with non-identified function were used as ligands. AutoDock Vina (Trott and Olson, 2010)was used to screen approximately 2 million commercially available small molecules filtered by "Lipinski's Rule of Five" (Lipinski et al., 2001). Target pocket for FAD and FBXL3 binding site was determined based on the CRY-FBXL3 crystal structure (Xing et al., 2013) .Autodock Tools4 or PyMol (*http:*//*pymol.sourceforge.net*/) software were used to visualize the docking results and protein structure, respectively.

A final number of 200 compounds with affinities ranging from -9 to -13.5 kcal/mol were then tested experimentally.

### Example 3 Cell Cytotoxicity Test

Human osteosarcoma U2-OS cell lines were used for the cytotoxicity assay. Cells were seeded in triplicate to clear 96-well plates with 4000 cells/well then grown for 48h. Cells were treated with molecules at desired concentrations (final DMSO concentration 0.5%) in DMEM. Cells were incubated with molecules for 48h. Cell viability was measured by adding tetrazolium dye 3-[4,5-dimethylthiazol-2-yl]-2,5 diphenyl tetrazolium bromide (MTT) which is converted to insoluble purple color formazan as a result of the mitochondrial activity. To measure the purple color after incubating cells with MTT reagent medium was replaced with DMSO:EtOH (50:50) mixture for 4 hours and absorbance of wells were measured at 570 nm by the spectrophotometer. Cells treated with 5% final DMSO concentration (known as toxic to cells) as negative controls.

### Example 4 Real-Time Monitoring of Circadian Rhythm

This assay was performed with two different equipment: Synergy H1 (BioTek) with 96-well plate and LumiCycle luminometer (Actimetrics) with 35mm plates. Initial screening of molecules to determine their effect on the circadian period was performed in 96-well plate via Synergy H1. Clear 35-mm plates are used in LumiCycle luminometer which provides high resolution data describing the circadian oscillation. For 96-plate tests, 50000 U2-OS Bmal1-dLuc cells were seeded on an opaque 96-well plate and cultured overnight. Next day cells were reset by adding dexamethasone (DXM) (0.1µM final) for 2h. Then medium is changed to bioluminescence recording media which contains the following in 1L: DMEM powder (sigma D-2902, 10X 1L), 0.35gr sodium bi-carbonate (tissue culture grade, sigma S5761), 3.5gr D (+) glucose powder (tissue culture grade, sigma G7021), 10mL 1M HEPES buffer (Gibco 15140-122), 2.5 mL Pen/Strep (100ug/ml), 50mL 5% FBS and up to 1L sterile milliQ water. Luciferin is added freshly with 0.1mM final concentration. Molecules were added to the bioluminescence recording media at desired concentration (0.5% final DMSO concentration). Plates were sealed with optically clear film to prevent evaporation and gas exchange thereby to maintain homeostasis of the cells.

Luminescence values were recorded at 32⁰C for each 30 minutes with 15 seconds integration time via Synergy H1 luminometer for a week. For LumiCycle, 400×10³ U2-OS Bmal1-dLuc or NIH3T3 mPer2-dLuc cells were seeded to 35mm plates and then procedure given above was followed with a change in the last step. Plates were sealed with vacuum grease and placed to LumiCycle. Each plate was recorded continuously every 10 minutes for 70 seconds at 37⁰C via photomultiplier tubes. Period and amplitude data were obtained from LumiCycle Analysis software. To detect the effect of FORMULA I in the absence of CRYs, Cry1^{-/-}/Cry2^{-/-} mouse embryonic fibroblasts (CRYDKO MEFs) transiently transfected with pGL3-Per2-Luc (luciferase reporter) were used. 3×10⁵ CRYDKO cells were seeded in 35mm clear plates. Next day, cells were transfected with 4000ng pGL3-Per2-Luc via Fugene6 transfection reagent according to the manufacturer's instruction. In short, 3:1 ratio of Fugene6 in µl against transfected DNA amount in µg was kept in transfections. 72h after transfection cells were synchronized with DXM for two hours. Then, medium was changed with lumicycle medium having DMSO or molecule, sealed with vacuum grease, and placed to LumiCycle.

### Example 5 CRY-LUC Cloning

To insert Cry1-Luc, Cry2-Luc constructs and only Luc into pcDNA4A-myc-his plasmid, coding sequence of mouse Cry1 and firefly Luciferase in pG5luc plasmid (Addgene) was amplified with primers having EcoRV-NotI and NotI-XhoI flanking sites for Crys and Luc, respectively. Product size was verified by visualizing in agarose gel and product was isolated from the gel by using NucleoSpin PCR and Gel purification kit (Macherey Nagel). pcDNA4A plasmid having Luc was double digested with NotI and XhoI and used as insert; Cry1 pcDNA4A-myc-his and Cry2 pcDNA4A-myc-his plasmids were double digested with NotI and XhoI FastDigest enzymes (Thermo Scientific) and used as host plasmids. Hosts were treated with FASTAP (Thermo Scientific) to prevent selfannealing. After gel isolation and cleaning inserts with Gel purification kit (Macherey Nagel) and destination vectors were ligated by using T4 DNA ligase (Thermo Scientific).

### Example 6 Site Directed Mutagenesis

Quick-change method was used for the site-directed mutagenesis. The primers were designed to have around 30 base pairs with the designated base changed in the middle of the primer. All the primer sequences for site-directed mutagenesis can be found. The PCR reaction mixtures contained 0.3 mM dNTP, 5 µl of 10X Phusion GC Buffer (Thermo Scientific), 3% DMSO, 1 µM of each primer, 50 ng of the template plasmid (mCry1 in pcDNA4A) and 1 unit of Phusion DNA polymerase in a 50 µl of final volume. The reaction conditions were set to 98⁰C for 30 seconds, 55⁰C for 30 seconds and 68⁰C for 5 minutes, 18 cycles. PCR products were visualized in 1% agarose gel. The samples with correct sized bands were digested with 1 unit of DpnI FastDigest enzyme (Thermo Scientific) for 1 hour at 37⁰C and then 5µl of sample was transformed to *E.coli* DH5α cells. Colonies were picked to culture and then plasmids were isolated via miniprep (Macherey Nagel). Sanger sequencing (Macrogen) was utilized to confirm the mutations.

### Example 7 CRY-LUC Degradation Assay

40ng of Cry1-Luc, Cry2-Luc, mutant Cry1-Luc plasmids or 5ng Luc plasmid were reverse transfected to 4×10⁴ HEK293T cells on opaque 96-well plate with flat bottom via PEI transfection reagent. 24h after transfection, cells were treated with molecules or solvent (DMSO). 24h of post molecule treatment cells were treated with luciferin (0.4mM final) and HEPES (10mM final and pH=7.2). After 2h, cycloheximide (20µg/ml final) was added to wells to stop protein synthesis. Plate was sealed with optically clear film and placed to Synergy H1. Luminescence readings were recorded every 10min at 32⁰C for 24 h. Half-life of protein was calculated via one-phase exponential decay fitting function in GraphPad Prism5 software. For each molecule or control at least three replicates were done in each experiment.

### Example 8 Protein immunoblot

Cells were lysed in RIPA buffer (50mM Tris, 150mM NaCl, 1% Triton-X, 0.1% SDS) with fresh Protease inhibitor cocktail (PIC). After centrifugation at 13000 rpm for 15 minutes at 4⁰C, supernatant was used to determine the protein amount using Pierce Protein Assay (Thermo Scientific) according to the manufacturer's instruction. Mice liver samples were homogenized and lysed with Dounce homogenizer and RIPA buffer. After homogenization samples were incubated 10 minutes on ice. After centrifugation 13000rpm 15 minutes at 4⁰C supernatant was used for the whole cell lysate. Subcellular fractionation of liver samples: Liver samples were homogenized with cytosolic lysis buffer (10mM HEPES pH 7.9, 10mM KCl, 0.1mM EDTA, 0.05% NP40, proteasome inhibitor) by pipetting 40 times with a cut tip. Then samples were incubated 10 minutes on ice and centrifuged for 3 minutes at 3000rpm at 4⁰C. Supernatant was saved in a new tube, and pellet was processed for the nuclear fraction. Supernatant was centrifuged at 13000 rpm for 3minutes at 4⁰C where new supernatant saved as the cytosolic fraction. Pellet was wash with cytosolic buffer and centrifuged 3 minutes at 3000rpm at 4⁰C and pellet was dissolved in nuclear lysis buffer (20mM HEPES pH 7.9, 0.4M NaCl, 1mM EDTA, 10%Glycerol, proteasome inhibitor), sonicated (60% power, 2 times 10 seconds x 3 cycles), and centrifuged 13000rpm for 5 minutes at 4⁰C. Supernatant is the nuclear fraction. After determining the protein amount, all lysates described above were supplemented with 4X Laemmli buffer (277.8mM Tris HCl pH: 6.8, 4.4% LDS, 44.4% (w/v) glycerol, 0.02% Bromophenol blue, (freshly added) 5% volume of beta Mercaptoethanol) and boiled at 950C for 10 minutes. Lysates mixed with Laemmli buffer were resolved in SDS-PAGE and transferred to PVDF membrane (Millipore). Membrane was blocked with 5% milk solution in 0.15%TBS- Tween for 1 hour and then incubated overnight with the following primary antibodies where they necessary: CRY1 (Bethyl Catalog No. A302-614A), CRY2 (Bethyl Catalog No. A302-615A), PER2 (Bethyl Catalog No. A303-109A), BMAL1 (Santa Cruz, sc-365645), anti-Myc (), anti-His (Santa Cruz SC-8036) BetaActin (Cell Signaling, 8H10D10), AlphaTubulin (Sigma, T9026), HistoneH3 (Abeam, ab1791). After washing membranes with 3 times for 5minutes with 0.15% TBS-Tween, membranes were incubated 1hour with appropriate HRP conjugated secondary mouse (Santa Cruz SC-358920) or rabbit (Cell Signaling, 7074) antibodies. ECL buffer system (Advansta WestemBright) was used to visualize HRP chemiluminescence via BioRad ChemiDoc Touch visualizer.

### Example 9 RNA Isolation and Real Time (RT) Quantitative PCR (qPCR)

QIAGEN RNeasy Mini Kit was used to isolate the mRNA. Protocol provided by the manufacturer was followed. On-column DNase treatment was performed with the DNase enzyme provided with the kit. Dounce homogenizer was used for the homogenization of the liver samples and followed protocol provided by the manufacturer to isolate RNA. Quantity of RNA was determined by Nanodrop 2000 (Thermo Scientific). Quality of RNA was verified by running in ~0.5% agarose gel prepared with DEPC water and visualizing under UV light. ~400ng of each RNA sample were subjected to first strand cDNA synthesis with MMLV-reverse transcriptase (Thermo) as the following: RNAs were mixed with 2µl Oligo(dT)23, 1µl 10mMdNTP mix, and nuclease-free H₂O to a final volume of 10µl (mix1). Mix1 was incubated at 65⁰C for 5 minutes to denature the possible secondary structures of RNA and primers which may prevent long cDNA synthesis then mixture was incubated at 4⁰C for 5 minutes. Mixture2 (mix2) containing 2 µl 10X reverse transcription buffer, 0.2µl Ribolock RNase inhibitor, 1µl Revert Aid Reverse Transciptase and 6.8µl nuclease-free H₂O was added to mix1. Mixture of mix1 and mix2 (totally 20µl) was incubated at 42⁰C for 1 hour and then at 65⁰C for 20 minutes to inactive the enzymatic activity. Volume of each sample was completed to 100µl with nuclease-free H₂O. For quantitative real time PCR (qRT-PCR) analysis cDNAs were further diluted 5-fold (1:5). mRNA expression levels were calculated by qRT-PCR with the SYBR Green. *Gapdh* gene was used as an internal control. A sample qRT-PCR reaction is the folowing: 8µl SYBR Green, 3µl cDNA (from 5-fold diluted solution), 1µl forward and reverse primer mix (0.5µM final), 8µl nuclease-free H₂O. All reactions were performed in biological triplicates (each triplicate with two technical replicates), and the results were expressed relative to the transcript level of *Gapdh* in each sample using the 2^{-ΔΔCT} method. qRT-PCR was run with the following cycling protocol: 95°C 10 seconds denaturation, 57 to 62⁰C for 20 seconds annealing (the degree is determined by primer type) and 72°C for 30 seconds of elongation, 35 cycles. List of primers used for the qRT-PCR can be found in Table 1 indicated as RT_GeneName.

**Table 1. Primers for the site-directed mutagenesis.**

| The red color indicates the mutated codon. | |
|---|---|
| Luc-pcDNA-NotI-F | GCACAGTGGCGGCCGCTCATGGAAGACGCCAAAAAC |
| Luc-pcDNA-XhoI-R | TCTAGACTCGAGCACGGCGATCTTTCC |
| mCRY1-pcDNA-EcoRV-F | TTCTGCAGATATCCAATGGGGGTGAACGCCGTG |
| mCRY1-pcDNA-NotI-R | AGACTCGAGCGGCCGCCAGTTACTGCTCTGCCGCTG |
| mCRY2-pcDNA-EcoRV-F | TTCTGCAGATATCCAATGGCGGCGGCTGCTGTG |
| mCRY2-pcDNA-NotI-R | GAGCGGCCGCCACTGTGCGGAGTCCTTGCTTGCTGG |
| mCRY1 W399L F | GCTGGAAGTTGGATGTTGCTGTCCTGCAGTTCC |
| mCRY1 W399L R | CGACCTTCAACCTACAACGACAGGACGTCAAGG |
| mCRY1 R293A F | CTTTATGGGCAACTCCTGTGGGCTGAATTTTTTTATACAGCAG |
| mCRY1 R293A R | CTGCTGTATAAAAAAATTCAGCCCACAGGAGTTGCCCATAAAG |
| | |
| RT_*hBMAL1_*F | GCCCATTGAACATCACGAGTAC |
| RT_*hBMAL1_*R | CCTGAGCCTGGCCTGATAGTAG |
| RT_*hCfZY1_*F | ACAGGTGGCGATTTTTGCTTC |
| RT_*hCRY1_*R | TCCAAAGGGCTCAGAATCATACT |
| RT_*hCRY2_*F | CTACCGGGGACTCTGTCTACT |
| RT_*hCRY2_*R | ACTGGGTAGTGGTCTTGGGC |
| RT_*hDBP_*F | GAGGAACTTAAGCCCCAGCC |
| RT_*hDBP_*R | CTCGTTGTTCTTGTACCGCC |
| RT_*hPER2_*F | GCGTGTTCCACAGTTTCACC |
| RT_*hPER2_*R | GGCTTTTCCGGACACTGACA |
| RT_*hGAPDH_*F | TGCACCACCAACTGCTTAGC |
| RT_*hGAPDH_*R | ACAGTCTTCTGGGTGGCAGTG |
| RT_*mGapdh_*F | AACTTTGGCATTGTGGAAGG |
| RT_*m^{g}apdh_*R | ACACATTGGGGGTAGGAACA |
| RT_*mPer2_*F | GAGCGCCACCAAGTGACGG |
| RT_*mPer2_*R | GGTGGGACTTGGGGAGAAGT |

### Example 10 CRY Repression Assay

4×10⁴ HEK 293T cells were reverse transfected with a mixture of 50 ng pSport6-Bmal1, 125ng pSport6-CLOCK, 50ng pGL3-mPer1::luc, 2.5ng or higher doses of pcDNA4A-Cry1 (WT or mutant) and empty Sport6 (to equalize the transfected amount of DNA totally 300ng) in a 96-well opaque plate via PEI transfection reagent. As a control same plasmid mixture without Cry1 was transfected to determine the activity of CLOCK/BMAL1 dependent transactivation in absence of the repressor. In each independent experiment transfections were done in triplicates for each condition. The plates were incubated for 24h at 37 °C, 5% CO2. Firefly luciferase and renilla luciferase expression was determined using the Dual-Glo Luciferase Assay System (Promega) using manufacturer's protocol.

### Example 11 Pull-down Assay with Biotinylated Molecule

10µg of Cry1-pcDNA4, Cry2-pcDNA4 or Cry1-T5-pcDNA4 plasmids expressing His and Myc at the C-terminal of CRYs were transfected to HEK293T cell. After 48h of transfection cells were harvested and lysed in lysis buffer (50 mM Tris pH 7.4, 2 mM EDTA, 1 mM MgCl2, 0.2% NP-40 (v/v), 0.1% sodium deoxycholate (w/v), 1 mM sodium orthovanadate, 1 mM sodium fluoride, and protease inhibitor cocktail (Thermo Scientific)). Lysates were incubated for 10 minutes on ice then mixed gently and incubated for another 10 minutes on ice. Then lysate was centrifuged for 10 minutes at 7000g at 4⁰C. 5% of the supernatant was kept for input analysis. Remaining supernatant was divided to three and mixed with 2x binding buffer (100 mM Tris-HCl pH 7.4, 300 mM NaCl, 0.2% NP-40 (v/v), 2mM sodium orthovanadate, 2 mM sodium fluoride, and protease inhibitor) with 1: 1 ratio, incubated with rotation having either of these: a) DMSO, b) biotinylated molecule, c) biotinylated molecule and the competitor for 2h. During this period NeutrAvidin Agarose resin (Thermo Scientific) was equilibrated with lysis buffer by mixing for 5 minutes via rotation at 4⁰C three times. After each mix period, resins were centrifuged at 2500g at 4⁰C for 2 minutes and supernatant was discarded. After 2h of mixing, lysates were incubated with equilibrated resins for 2h to isolate the proteins interacting with the molecule. After 2h, resins were centrifuged (2500g, 2 minutes, 4⁰C) supernatants were discarded and washed with 1× binding buffer six times. Finally, proteins bound to resin were isolated by adding 50µl 4X Laemmli buffer and boiling at 95⁰C for 10 minutes. Pulled-down samples were analyzed via western blot.

### Example 12 In Vivo Mice Studies

### Animals and their synchronization

Male and female C57BL/6J mice, 8-12 weeks of age, weighing 18-24 g were used in the in vivo studies with FORMULA I. Mice were obtained from Koç University Animal Research Facility (KUTTAM) and experiments were conducted in accordance with the guidelines approved for animal experimental procedures by the Koc University Animal Research Local Ethics Committee (No: 2015/13).

Mice were housed in polystyrene cages up to four animals in the room equipped with temperature control (21 ± 2°C) and humidity (55 ± 5%). Mice were housed under 12 h of light (L) in alternation with 12 h of darkness (D) (LD 12:12) prior to any intervention and the same lighting regimen continued to the end of the experiment. Water and food were provided ad libitum throughout the experiments. FORMULA I or vehicle were administered intraperitoneally to mice at the same time every day (three hours after light onset).

### Preparation of FORMULA I formulation

FORMULA I was dissolved in 2.5% DMSO and 15% Cremophor EL and then diluted with 82.5% isotonic sodium chloride solution (Vehicle= DMSO:Cremophor EL:0.9% NaCl; 2.5:15:82.5, v/v/v) on each study day to prepare freshly, prior to intraperitoneal injection. Solvents were reagent grade, and all other commercially available reagents were used as received unless otherwise stated.

### Determination of the Dose Range

The dose levels to be used in the single dose toxicity study were selected according to the OECD Guidelines (Guidelines for the testing of chemicals, 2002). Male (n=2-3) and female (n=2-3) C57BL/6J mice were used at each dose level. Mice were treated with 5, 50, 300 or 1000 mg/kg single doses of FORMULA I intraperitoneally, one dose being used per group. Control mice (n=3 for both sexes) were only treated with vehicle (DMSO:Cremophor EL:0.9% NaCl; 2.5:15:82.5, v/v, i.p.). Careful observations of mice including body weight changes, body temperature, behavioral and clinical abnormality, and mortality were performed during 14 days, and gross necropsy of all animals was carried out at the end of the experiment. Body weight was measured every day as an index of general toxicity. FORMULA I -induced body weight change was expressed relative to body weight on the initial treatment day. The body temperatures of the mice were recorded by rectal homeothermic monitor (Harvard Apparatus, US) for 5 days following FORMULA I injection. Temperature measurements were performed at the same time each day. Food intake and water consumption were also monitored for 14 days. After the observation period, mice were exposed to isoflurane anesthesia and blood was collected by cardiac puncture. Mice were immediately sacrificed by cervical dislocation after blood collection. Hematological parameters were analyzed in blood samples.

### Repeated Dose Toxicity Study: Determination of the Maximum Tolerated Dose of FORMULA I Upon 5-Day Administration

The single dose toxicity data were used to assist the selection of the doses in this repeated dose toxicity study. C57BL/6J mice (n=6 per group) were treated with 40, 80 or 150 mg/kg doses of FORMULA I intraperitoneally for 5 days. Control mice (n=4) were only treated with vehicle (DMSO:Cremophor EL:0.9% NaCl; 2.5:15:82.5, v/v, i.p.). Throughout the study, animals were monitored for mortality, clinical signs, body weight changes, body temperature, food and water consumptions, behaviour assessment and gross findings at the terminal necropsy. Body weights and body temperatures of mice were measured every day as an index of toxicity. Within 48h after the last dose of FORMULA I administration, blood was collected from mice by cardiac puncture under isoflurane anesthesia. Hematological and biochemical analyzes were performed in blood. Liver, spleen, kidneys and lungs were removed and fixed in 10% formalin solution for histological examinations.

### Repeated Dose Toxicity Study: Determining the Subacute Toxicity of 60 mg/kg FORMULA I Upon 14-Day Administration

According to the results of the 5-day maximum tolerated dose determination study, FORMULA I dose was determined as 60 mg/kg for the next 14-day subacute toxicity study. In this study, C57BL/6J mice (n=10) were treated with 60 mg/kg dose of FORMULA I intraperitoneally for 14 days. Control mice (n=5) were only treated with vehicle. Following this, aforementioned procedure (5-day maximum tolerated dose determination study) has been conducted in the same way.

### Pharmacokinetic Studies

C57BL/6J female mice (n=4 per each time point) were treated with 100 mg/kg single dose of FORMULA I intraperitoneally. Blood samples were collected 0, 0.5, 1, 2, 4, 8, 12 and 24h after administration of FORMULA I by cardiac puncture under isoflurane anesthesia. Plasma was obtained by centrifugation from heparinized tubes and stored at -80 °C until analysis. Brain tissues were quickly removed and stored at -80 °C for further processing. For assessment of the brain exposure of FORMULA I, levels in this tissue were only determined at 2nd (n=2) and 4th (n=2) hours according to maximum concentrations of the molecule in plasma.

### Determination of FORMULA I Levels in Plasma and Brain

Liquid chromatography coupled to tandem mass spectrometry (LC-MS/MS) was used to obtain high mass accuracy data of the analytes in plasma samples for the determination of FORMULA I and Internal standart (IS) Loratadine. The instrument was operated in full scan mode and ion source parameters were optimised for the analytes. The optimized conditions of the electrospray ionization (ESI) source were as follows: gas temperature, 300°C; gas flow, 10 L/min; nebulizer pressure, 25 psi; capillary voltage, 5500 V. ESI in the positive ion mode provides the best results for FORMULA I . For MS-MS detection, the protonated molecules were selected as precursor ions and the most abundant fragment ions obtained at collision energy of 22-20 eV were monitored as the product ions for FORMULA I and IS. MS-MS analysis was performed in the selected reaction monitoring (SRM) positive ionization mode, using mass transitions for FORMULA I m/z 472.1 - 353.4 [collision energy (CE) = 22 eV] and Fragmentor 234 V, for IS; m/z 383.2→ 337.2 (CE = 20 eV) and fragmentor 140 V. For the separation of the analytes, a conventional reversed-phase LC separation on C18-bonded silica was used, with Methanol: % 0.1 Formic acid (90:10, v/v) mobile phase. FORMULA I was extracted from plasma samples by using liquidliquid extraction with Methanol. 10 µL of sample was injected into the LC-MS/MS system.

### Pharmacokinetic Analysis

Peak plasma concentration (Cmax) and time to reach peak plasma concentration (tmax) values were directly obtained from the FORMULA I plasma concentration-time curve. The area under the plasma concentration-time curve from 0 to 48h (AUC0-48h) was calculated by the trapezoidal method. AUC0-∞ was obtained by addition of the extrapolated part after the last sampling time using standard techniques. Other pharmacokinetic parameters of FORMULA I were calculated by the non- compartmental method. Elimination rate constant (kel) was calculated from the terminal points of FORMULA I plasma concentration-time plot and the slope of this line was equal to kel. Terminal elimination half-life (t1/2) was calculated by ln-linear approximation of terminal points of the data. t1/2 and kel were interconverted with the following formula: t_{1/2} = ln2 / kₑₗ

### Circadian Behavioral Analysis

Mice were housed in individual cages within a temperature- and humiditycontrolled, light-tight enclosure. Each cage contained a running wheel. Food and water were allowed *ad libitum.* Animals were entrained to a 12:12 h L:D cycle for ≥2 weeks before being released into constant darkness. Then, vehicle (*n*=5) and FORMULA I (n=6) administered to animals for 5 days and then animals were kept under constant condidions for more than 2 weeks. Locomotor activity monitoring, actogram creation, and period calculations were performed using ClockLab Data Collection (Actimetrics). Statistical analysis of period change was done through application of both a t test and an alternative, nonparametric Mann-Whitney test using the t.test() and wilcox.test() functions in R. Both tests resulted in a significantly (*p*≤.05) greater period among mutant as compared to wild-type mice.

### Example 13 Statistical Analysis

All data were expressed as means ± standard error of the means (SEM) for each studied variable. Statistical analyses were performed using GraphPad Prism for Windows (GraphPad Software, California, USA). The statistical significance of differences between groups was validated with either of these: Student's t-test and one- or two-way analysis of variance (ANOVA), following Tukey or Bonferroni post hoc tests, respectively. Type of significant test used in each experiment and their p-values were indicated in the figure legends.

### Results

### Identification of the Molecules Effect the Half-Life of the CRY

Initially, toxicity of selected molecules was assessed by MTT using immortalized human osteosarcoma (U2-OS) cells at concentrations of 20 µM, 10 µM and, 2.5 µM. Molecules with relative cell viability less than 90% at 2.5 µM were eliminated. Next, the effects of the nontoxic molecules on circadian rhythm were assed using U2-OS cells stably expressing Bmal1- luc construct. Analysis of the results indicated 14 molecules affect the circadian rhythm either by increasing or decreasing the period by1 hour or more. In order to find molecules that either enhance or decrease half-life CRY, we used CRY1::LUC degradation assay where CRYlwas fused with Luciferase (LUC) at the C-terminal. In this assay the effect of the 14 molecules were measured according to the CRY1::LUC decay rate. After transfection of HEK293T cells with CRY1-LUC plasmids, cells were treated with these non-toxic molecules. Then cells were treated with cycloheximide (CHX) to inhibit the protein synthesis and bioluminescence was recorded for 18h. Of these 14 molecules, 6 of them significantly decreased the half-life of CRY1- LUC. We focused on the molecules that destabilized CRY1 and tested their dose- dependent effect on the circadian rhythm of U2-OS Bmal1-dLuc cells. Among these 6 molecules, FORMULA I showed the best dose responsive effect. Thus FORMULA I was selected for further characterization.

### Characterization of FORMULA I

Before we begin the characterization of the FORMULA I on circadian clock we first assed it effect on half-life on LUC and its toxicity on U2-OS cells in detail. FORMULA I had no effect on the stability of LUC and any toxic effect in dose dependent manner. We then determined the effect of FORMULA I on circadian rhythm using U2-OS Bmal1-dLuc cells and NIH 3T3 Per2-dLuc cells in a dose dependent manner. FORMULA I significantly lengthened the period and dampened the rhythm a dose-dependent manner with the EC50 value of ~6.5µM in U2-OS (Fig 1B). FORMULA I treated NIH 3T3 cells exhibited similar phenotype in dose dependent manner. To eliminate the possibility that FORMULA I might affect the other proteins and, in turn, circadian rhythmicity we tested the effect of FORMULA I in Cry1^{-/-}Cry2^{-/-} mouse embryonic fibroblast cells (DKO- MEF) transfected with mPer2-luc plasmid. Results indicated that FORMULA I had no effect on was through CRY (Fig. 1C). We next determined the effect of the FORMULA I on the half-of both CRY1 an CRY2 by measuring the decay rate of CRY1-LUC and CRY2-LUC. To this end HEK 293T cells were transfected with Cry-Luc plasmids. Cells were, then, treated with cycloheximide to inhibit protein translation in the presences of the various amount of FORMULA I . Notably, FORMULA I reduces the half-life of CRY1 in dose dependent manner while there was no effect on the half-life of CRY2 (Fig. 2 A,B). Next, unsynchronized U2-OS cells were treated with FORMULA I and the level of both CRY1 and CRY2 level were measured by Western blot. Consistent with our CRY-LUC assay, the level of CRY1 reduced while the CRY2 levels were comparable between FORMULA I treated cells and DMSO-treated cells (Fig.2C) FORMULA I binds to CRY1 through R293 and W399 amino acid residues identified by Autodock Vina (Fig. 2D). Docking FORMULA I to CRY1 showed that indoline groups of FORMULA I and Trp399 interact through a strong pi-pi interaction. In addition, benzene group at the other side of the FORMULA I interacts with Arg293 through a pi-cation interaction (Fig. 2D). The replacement of these residues in CRY1 eliminates the binding of FORMULA I to CRY1. To test this, R293 and W399 of CRY1 were replaced with Ala and Leu by site directed mutagenesis, respectively. Before measuring the effect of FORMULA I on this CRY1 mutant, we confirmed CRY-1R293AW399L mutant retained its repressor activity with Per1-Luc assay using BMAL1/CLOCK in the presences of the mutant and wild type CRY1. We then measured half-life CRY1R293AW399L: :LUC. FORMULA I had no effect on the half-life of CRY1-R293A-W399L compared to control (Fig. 2E). This approved that FORMULA I binds the computationally predicted region.

All these results showed FORMULA I binds to the FAD binding pocket in the PHR domain and specifically destabilizes CRY1.

### Physical Interaction between FORMULA I and CRY1

To show the physical binding of the FORMULA I to CRY1, biotinylated-FORMULA I (bFORMULA I ) was commercially synthesized by Enamine (Ukraine). To test whether bFORMULA I binds CRY1 plasmids have His-Myc tagged Cry 1 and His-Myc tagged Cry2 were transfect to HEK293T cells. After preparation of the cell lysate, bFORMULA I was used to pull-down CRY1-HIS-MYC and CRY2-HIS-MYC in the presence and absence of the competitor (FORMULA I ). Results indicated that FORMULA I physically binds to CRY1 but not to CRY2 (Fig. 2F). Binding of bFORMULA I to CRY1 disappeared in the presence of the free FORMULA I . To confirm the binding of the molecule to PHR domain of the CRY1 we performed a pull down assay between bFORMULA I and CRY1-T5 (the lack of 100 amino acids from C-terminal end). Result showed FORMULA I bound to the PHR domain (Fig. 2G). Furthermore, the activity of biotinylated FORMULA I was confirmed on circadian rhythm and half-life of CRY1 to make sure that biotinylation did not alter the function and binding of the molecule. Pull down assays along with mutagenesis studies suggested that FORMULA I physically binds to CRY1 through the FAD binding pocket.

To understand why CRY2 was unable to bind FORMULA I we performed computational studies on FAD binding region of the CRY2. We ran a 10 ns simulation of mouse CRY2 (mCRY2) (PDB ID: 4I6G) and used it for docking FORMULA I . FORMULA I could not interact with the residues W417 and R311 in CRY2, which are homologous to W399 and R293 of CRY1 (Fig. 2H). Thus, Autodock Vina predicted the binding energy of FORMULA I to CRY2 as -9.5 kcal/mol while it predicted that of CRY1 as -13.3 kcal/mol. To further understand the difference in the binding residues of FORMULA I between CRY1 and CRY2, we analyzed the equilibrated CRY1 and CRY2 structures utilized in docking simulations. Although residues in PHR of CRY1 and CRY2 are 77% identical, 88% similar, two of the critical residues (W399 in CRY1, W417 in CRY2; W292 in CRY1, W310 in CRY2) locate themselves differently in equilibrated CRY1 and CRY2 structures. To understand the conformational changes atoms were measured throughout the simulations. Dihedral angle around 500 for W399 CRY1 (W417 in CRY2) corresponds to conformation where tryptophan residue is parallel to R293 in CRY1 (R311 in CRY2) where values around 1000 corresponds to parallel position. Dihedral value around -1500 for W292 in CRY1 (W310 in CRY2) corresponds to parallel position of this residue with respect to R293 in CRY1 (R311 in CRY2), however >00 corresponds to perpendicular position (Fig. 2I). While side chains of W399 and W292 were mostly located parallel to R293 in CRY1 and left enough space FORMULA I to bind, W417 and W310 in CRY2 acquired a position perpendicular to R311 where they occupied most of the FORMULA I binding space in CRY2. Although W399 mainly maintained its position parallel to R293 in CRY1 (76% of the entire simulation), W310 kept its position perpendicular to R311 in CRY2 and filled the binding space of FORMULA I through the entire simulation. Superimposing the equilibrated structures of CRY1 and CRY2 showed clearly how these critical amino acid residues were located differently (Fig. 2K). As a result, parallel position of W399 and W292 allowed binding of FORMULA I t o CRY1. However, perpendicular position of W417 and W310 to R311 precluded FORMULA I binding to CRY2. Similar results were verified from 50 ns independent simulations for both CRY1 and CRY2.

These results showed that the internal dynamics of CRY1 and CRY2 are different even at the very conserved region which can modulate their interactions with proteins or ligands, and thus their cellular roles.

### Time-Dependent Effect of FORMULA I on the U2-OS Cells

To evaluate the effect of FORMULA I on the endogenous protein and mRNA level of clock genes, synchronized U2-OS cells were treated with 10 µM FORMULA I . Cells were harvested between 72-92 hours of post-synchronization at 4h time intervals. FORMULA I decreased the level of CRY1, especially between 80-92h (Fig. 3A). On the other hand, we observed increased CRY2 levels which is most likely to compensates for the reduction of CRY1. Analysis of mRNA level of *Cry1* and *Cry2* showed that FORMULA I changed the peak time without significantly changing their overall abundance (Fig. 3B).

Increase in clock output genes of the Dbp and Per2 mRNA levels also confirmed the decrease in the total repressor capacity of the cells treated with FORMULA I . Decreased mRNA and protein levels of BMAL1 indicates that FORMULA I did not affect BMAL1 post-translationally. All these results suggest that FORMULA I is a promising molecule to regulate circadian clock machinery through CRY1. To investigate pharmacological properties and activity of FORMULA I in vivo, it has been subjected to in mice studies.

### Single, Repeated and Subactute FORMULA I Toxicity Studies in Mice

To evaluate the toxicity of the FORMULA I in vivo we first performed single dose toxicity study (SDT). FORMULA I was intraperitoneally (i.p.) administered to C57BL/6J mice at the doses of 5, 50, 300 and 1000 mg/kg. General toxicity was evaluated on the basis of mortality, body weight changes, body temperature, clinical signs, food and water consumptions, behavior assessment and gross findings at terminal necropsy. Animals treated with 1000 mg/kg of the FORMULA I e xhibited the following clinical symptoms: dyspnea, hyporeflexia, reduced locomotor activity, piloerection, hunched posture and corneal opacity. This dose was considered lethal and animals were scarified for the ethical reasons. No mortality and clinical signs were observed in other doses (5, 50, 300 mg/kg). Compared to control mice (treated with vehicle where their body temperatures were 36.0-37.0_{°}C) mild hypothermia (33.5-35.1_{°}C) was observed in mice within 6h after administrating the FORMULA I and body temperatures gradually increased in the next hours at the dose of 300 mg/kg (Fig. 4A). The body temperature of the animals were comparable to control groups at the dose of 5 mg/kg while the body temperatures of the animals were higher in animals treated with 50 mg/kg (Fig.4A). We then measured weight loss on these animals for 15 days. The administration (Fig 4B). There were no significant weight losses in animals treated with 5 mg/kg of the FORMULA I . All these result suggested that STD of 5 and 50 mg/kg were well-tolerated by mice.

We next determined the 5-day maximum tolerated dose (MTD) using 40, 80 or 150 mg/kg of FORMULA I with repeated injections for 5 days. A single death (1/6; 16.6%) among all tested doses was observed in the group of 150 mg/kg on the 3th day of injection. There were no observed clinical signs for the animals treated with 40 and 80 mg/kg of the FORMULA I . The following clinical signs were observed in animals treated with 150 mg/kg of the FORMULA I hyporeflexia, which were more prominent in the first three days of treatment and lightened on 4th and 5th day. The body temperature of the animals treated with 40 mg/kg of FORMULA I was comparable to the control animals. On the other hand mild hypothermia (33.4-35.8_{°}C) was measured in mice on the first day at doses of 80 and 150 mg/kg. Body temperatures increased gradually in the following days (Fig. 4C). The body weight losses were similar for all doses with exception at 3rd and 4th injections at the dose of 150 mg/kg where the highest mean body weight loss (6.3%) was observed on the 3rd day of injection (Fig. 4D).All these results suggested that doses of 5 and 80 mg/kg were well-tolerated by mice. We therefore decided to carry out subacute toxicity test to further evaluate the effect of the FORMULA I on animals using dose of 60 mg/kg for 14-days of repeated injections.

When FORMULA I was administered to animals once a day for 14 days no mortality and clinical signs were observed for the 60 mg/kg dose. Mild hypothermia (33.7-35.4_{°}C) was recorded in mice during injections (Fig. 4E). The highest mean body weight loss (3.7%) occurred on the first day following administration and body weights increased through the next 13 days of injections (Fig. 4F).

### Determination of Pharmacokinetic Profile of FORMULA I

Mean plasma concentration-time curve of FORMULA I administered at 100 mg/kg single dose (i.p.) to female C57BL/6J mice were presented in Fig 4G, and pharmacokinetic parameters of FORMULA I were given in Table 2.

**Table 2. Plasma pharmacokinetic parameters of FORMULA I (100 mg/kg, single dose, i.p.)**

| Parameters | Values |
|---|---|
| Cₘₐₓ (ng/ml) | 1150.52±506.26 |
| tₘₐₓ (h) | 2-4 |
| AUC₀₋₂₄ (ng.h/ml) | 4921±3069.09 |
| AUC_{0-∞} (ng.h/ml) | 5194±3134.00 |
| kₑₗ (1/h) | 0.127±0.01 |
| t_{1/2} (h) | 5.5±0.33 |

FORMULA I plasma level was first detected at 0.5h, reached maximum level at 2-4h and was still quantifiable at 24h using HPLC and Mass spectrophotometry. For the assessment of the brain exposure of FORMULA I, levels in this tissue were only determined at 2nd (n=2) and 4th (n=2) hours according to maximum concentrations of the molecule in plasma. FORMULA I molecule was detected in the brain tissue (Fig. 4H). All these results suggested that FORMULA I molecule has half-life in vivo and crosses the blood-brain barrier.

### In vivo Effect of FORMULA I

All in vitro studies suggested that FORMULA I binds and reduces half-life of CRY1. To assess its in vivo effect, FORMULA I was intraperitoneally (i.p.) administered into mouse at 25mg/kg and 50 mg/kg doses. Mice were sacrificed 6h after the injection and all organs were kept for downstream analysis. We could observe a slight decrease in CRY1 levels in the whole cell lysate of mouse liver cells (Fig 5A).

Since CRYs are stabilized and degraded differently in cytosolic and nuclear compartments, we further analyzed the level of CRY1 in the cytosolic and nuclear fraction of the mouse liver. We fractionated the same liver samples and separately isolated cytosolic and nuclear proteins. Proteins, known to be specifically localized in nucleus (Histone-H3) and cytoplasm (Tubulin), were used as controls to evaluate the purity of the fractions. CRY1 abundance in nucleus was significantly low compared to controls in dose dependent manner (Fig. 5B). On the other hand, cytosolic levels of CRY1 in FORMULA I treated mice liver were not statistically significant compared to controls (Fig. 5C). To further explore the influence of FORMULA I on circadian transcriptional function in vivo, we used qPCR to measure the transcriptional level of Per2. The Per2 gene level significantly increased in mice treated with FORMULA I (Fig. 5D). All these suggested that FORMULA I is effective on CRY 1levels in mouse liver cells.

### Effect of FORMULA I on Apoptosis in p53^{-/}⁻Mouse Embryonic Fibroblast Cells

In previous studies it was reported that cytochrome mutation sensitizes the Ras transformed p53-null cells, but not cells with wild-type p53, to apoptosis induced by UV or UV mimetic agents e.g oxaliplatin. Using fibroblasts isolated from the skin of *Cry1*^{*-*/}*⁻Cry2^{-l-}p53*^{*-*/*-*} and *p53*^{*-*/*-*} mice, it has been shown that Cry deletion on the *p53*-null background sensitized the cells to bulky-DNA adduct-induced apoptosis (Lee and Sancar, 2011). With the expectation that FORMULA I would destabilize CRY1 and increase the effectiveness of UV mimetic chemotherapeutic agents, we performed apoptosis assay with Ras transformed *p53*-null MEF cell lines. In this assay, cells were treated by increasing dose of oxaliplatin for 16h followed by FORMULA I treatment for 24h and probed for apoptosis by measuring PARP cleavage. As chemotherapeutic agent, oxaliplatin was preferred since mutation of Cry sensitizes *p53*-null cells to apoptosis (Lee et al., 2013). We found that FORMULA I treatment sensitized Ras transformed p53-null MEF cell line to oxaliplatin-induced apoptosis measuring indicators of apoptosis cleaved PARP in dose dependent manner (Fig. 6).

### Effect of FORMULA I on Apoptosis in p53^{-/}⁻Mouse Embryonic Fibroblast Cells

To investigate the effect of the Formula I on the behavior of mice, animals are treat with Formula I under constant dark conditions. As can be seen in Fig. 7, animals treated with Formula I had approximately 14 min shorter period length compared with animals treated with vehicle. We next determined the amplitude of the animals. Formula 1 had statistically significant impact on the amplitude compared with control animals (Fig. 7D).

### References

Chen, Z., Yoo, S.H., Park, Y.S., Kim, K.H., Wei, S., Buhr, E., Ye, Z.Y., Pan, H.L., and Takahashi, J.S. (2012). Identification of diverse modulators of central and peripheral circadian clocks by high-throughput chemical screening. P Natl Acad Sci USA 109, 101-106.
Chun, S.K., Jang, J., Chung, S., Yun, H., Kim, N.J., Jung, J.W., Son, G.H., Suh, Y.G., and Kim, K. (2014). Identification and validation of cryptochrome inhibitors that modulate the molecular circadian clock. ACS Chem Biol 9, 703-710.
Czama, A., Berndt, A., Singh, H.R., Grudziecki, A., Ladurner, A.G., Timinszky, G., Kramer, A., and Wolf, E. (2013). Structures of Drosophila Cryptochrome and Mouse Cryptochrome1 Provide Insight into Circadian Function. Cell 153, 1394-1405.
Hirano, A., Yumimoto, K., Tsunematsu, R., Matsumoto, M., Oyama, M., Kozuka-Hata, H., Nakagawa, T., Lanjakornsiripan, D., Nakayama, Keiichi I., and Fukada, Y. (2013). FBXL21 Regulates Oscillation of the Circadian Clock through Ubiquitination and Stabilization of Cryptochromes. Cell 152, 1106-1118.
Hirota, T., Lee, J.W., St John, P.C., Sawa, M., Iwaisako, K., Noguchi, T., Pongsawakul, P.Y., Sonntag, T., Welsh, D.K., Brenner, D.A., et al. (2012). Identification of Small Molecule Activators of Cryptochrome. Science 337, 1094-1097.
Hirota, T., Lewis, W., Liu, A., Lee, J., Schultz, P., and Kay, S. (2008). A chemical biology approach reveals period shortening of the mammalian circadian clock by specific inhibition of GSK-3beta. Proc Natl Acad Sci USA 105, 20746-20751.
Khan, S.K., Xu, H., Ukai-Tadenuma, M., Burton, B., Wang, Y., Ueda, H.R., and Liu, A.C. (2012). Identification of a Novel Cryptochrome Differentiating Domain Required for Feedback Repression in Circadian Clock Function. Journal of Biological Chemistry 287, 25917-25926.
Lampson, M.A., and Kapoor, T.M. (2006). Targeting protein stability with a small molecule. Cell 126, 827-829.
Lee, J.H., Gaddameedhi, S., Ozturk, N., Ye, R., and Sancar, A. (2013). DNA Damage-Specific Control of Cell Death by Cryptochrome in p53-Mutant Ras-Transformed Cells. Cancer Research 73, 785.
Lee, J.H., and Sancar, A. (2011). Circadian clock disruption improves the efficacy of chemotherapy through p73-mediated apoptosis. Proceedings of the National Academy of Sciences 108, 10668.
Lin, C., and Todo, T. (2005). The cryptochromes. Genome Biol 6, 220.
Lipinski, C.A., Lombardo, F., Dominy, B.W., and Feeney, P.J. (2001). Experimental and computational approaches to estimate solubility and permeability in drug discovery and development settings. Adv Drug Deliv Rev 46, 3-26.
Morris, G.M., Huey, R., Lindstrom, W., Sanner, M.F., Belew, R.K., Goodsell, D.S., and Olson, A.J. (2009). AutoDock4 and AutoDockTools4: Automated docking with selective receptor flexibility. J Comput Chem 30, 2785-2791.
Oda, A., Katayose, Y.U., Yabuuchi, S., Yamamoto, K., Mizuma, M., Shirasou, S., Onogawa, T., Ohtsuka, H., Yoshida, H., Hayashi, H., et al. (2009). Clock Gene Mouse Period2 Overexpression Inhibits Growth of Human Pancreatic Cancer Cells and Has Synergistic Effect with Cisplatin. Anticancer Research 29, 1201-1209.
Oshima, T., Yamanaka, I., Kumar, A., Yamaguchi, J., Nishiwaki-Ohkawa, T., Muto, K., Kawamura, R., Hirota, T., Yagita, K., Irle, S., et al. (2015). C-H activation generates period-shortening molecules that target cryptochrome in the mammalian circadian clock. Angew Chem Int Ed Engl 54, 7193-7197.
Ozturk, N., Lee, J.H., Gaddameedhi, S., and Sancar, A. (2009). Loss of cryptochrome reduces cancer risk in <em>p53</em> mutant mice. Proceedings of the National Academy of Sciences 106, 2841.
Schmalen, I., Reischl, S., Wallach, T., Klemz, R., Grudziecki, A., Prabu, J.R., Benda, C., Kramer, A., and Wolf, E. (2014). Interaction of Circadian Clock Proteins CRY1 and PER2 Is Modulated by Zinc Binding and Disulfide Bond Formation. Cell 157, 1203-1215.
Solt, L.A., Wang, Y.J., Banerjee, S., Hughes, T., Kojetin, D.J., Lundasen, T., Shin, Y., Liu, J., Cameron, M.D., Noel, R., et al. (2012). Regulation of circadian behaviour and metabolism by synthetic REV-ERB agonists. Nature 485, 62-68.
Trott, O., and Olson, A.J. (2010). AutoDock Vina: improving the speed and accuracy of docking with a new scoring function, efficient optimization, and multithreading. J Comput Chem 31, 455-461.
Xing, W.M., Busino, L., Hinds, T.R., Marionni, S.T., Saifee, N.H., Bush, M.F., Pagano, M., and Zheng, N. (2013). SCFFBXL3 ubiquitin ligase targets cryptochromes at their cofactor pocket. Nature 496, 64-+.
Yoo, S.H., Mohawk, J.A., Siepka, S.M., Shan, Y., Huh, S.K., Hong, H.K., Kornblum, I., Kumar, V., Koike, N., Xu, M., et al. (2013). Competing E3 ubiquitin ligases govern circadian periodicity by degradation of CRY in nucleus and cytoplasm. Cell 152, 1091-1105.

## Claims

1. The use of a compound having the following formula or a pharmaceutically acceptable salt thereof, for the preparation of a medicament for treatment and/or prevention of diseases or disorders associated with the circadian rhythm,

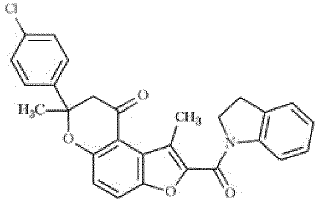

2. Use of a compound according to claim 1 for the preparation of a medicament useful in the treatment and/or prevention of diseases or disorders related to CRY1.

3. Use of a compound according to claim 2, wherein the disease or disorder is cancer.

4. Use of a compound according to claim 1, wherein the disease or disorder is sleep disorders such as delayed sleep phase syndrome.

5. Use of a compound according to claim 1, wherein the disease or disorder is related with circadian amplitude reduction.

6. A pharmaceutical composition comprising; a pharmaceutical carrier and
- a therapeutically effective amount of a compound having the following formula or a pharmaceutically acceptable salt thereof,

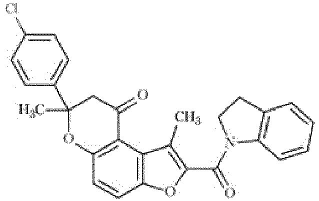

## Patentansprüche

1. Verwendung einer Verbindung der folgenden Formel oder eines pharmazeutisch akzeptablen Salzes davon zur Herstellung eines Arzneimittels für die Behandlung und/oder Vorbeugung von Krankheiten oder Störungen, die mit dem zirkadianen Rhythmus verbunden sind,

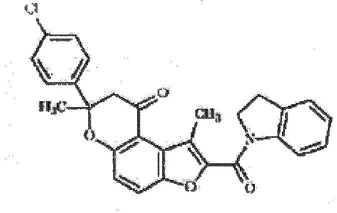

2. Verwendung einer Verbindung nach Anspruch 1 zur Herstellung eines Arzneimittels, das für die Behandlung und/oder Vorbeugung von Krankheiten oder Störungen, die mit CRY1 verbunden sind, nützlich ist.

3. Verwendung einer Verbindung nach Anspruch 2, wobei es sich bei der Krankheit oder Störung um Krebs handelt.

4. Verwendung einer Verbindung nach Anspruch 1, wobei es sich bei der Krankheit oder Störung um Schlafstörungen wie das Syndrom der verzögerten Schlafphase handelt.

5. Verwendung einer Verbindung nach Anspruch 1, wobei die Krankheit oder Störung mit einer Verringerung der zirkadianen Amplitude verbunden ist.

6. Eine pharmazeutische Zusammensetzung umfassend; einen pharmazeutischen Träger und
- eine therapeutisch wirksame Menge einer Verbindung der folgenden Formel oder ein pharmazeutisch akzeptables Salz davon,

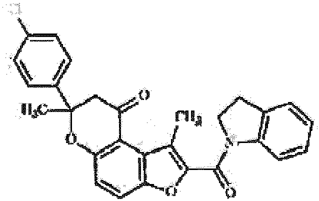

## Revendications

1. Utilisation d'un composé ayant la formule suivante ou d'un sel pharmaceutiquement acceptable de celle-ci, pour la préparation d'un médicament destiné au traitement et/ou prévention de maladies ou troubles associés au rythme circadien ;

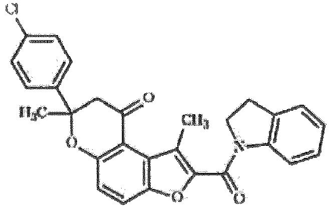

2. Utilisation d'un composé selon la revendication 1 pour préparation d'un médicament utile pour le traitement et/ou prévention des maladies ou troubles ou se rapportant au CRY1.

3. Utilisation d'un composé selon la revendication 2, dans lequel la maladie ou le trouble est le cancer.

4. Utilisation d'un composé selon la revendication 1, dans lequel la maladie ou le trouble est un trouble du sommeil tel que le syndrome de retard de phase du sommeil.

5. Utilisation d'un composé selon la revendication 1, dans lequel la maladie ou le trouble est lié à la réduction de l'amplitude circadienne.

6. Une composition pharmaceutique comprenant ; un véhicule pharmaceutique et
- une quantité thérapeutiquement efficace d'un composé ayant la formule suivante ou d'un sel pharmaceutiquement acceptable de celle-ci,
